(19) Europäisches Patentamt
European Patent Office
Office européen des brevets

(11) Veröffentlichungsnummer: **0 342 569 B1**

# (12) EUROPÄISCHE PATENTSCHRIFT

(45) Veröffentlichungstag der Patentschrift: **22.06.94**

(51) Int. Cl.[5]: **C07D 239/42**, C07D 239/47, C07D 239/52, C07D 251/16, C07D 251/52, C07D 251/46, A01N 47/36

(21) Anmeldenummer: **89108702.5**

(22) Anmeldetag: **16.05.89**

Die Akte enthält technische Angaben, die nach dem Eingang der Anmeldung eingereicht wurden und die nicht in dieser Patentschrift enthalten sind.

## (54) Heterocyclische 2-Alkoxyphenoxysulfonylharnstoffe und ihre Verwendung als Herbizide oder Pflanzenwachstumsregulatoren.

(30) Priorität: **17.05.88 DE 3816704**

(43) Veröffentlichungstag der Anmeldung: **23.11.89 Patentblatt 89/47**

(45) Bekanntmachung des Hinweises auf die Patenterteilung: **22.06.94 Patentblatt 94/25**

(84) Benannte Vertragsstaaten: **BE CH DE ES FR GB IT LI NL SE**

(56) Entgegenhaltungen:
**EP-A- 0 004 163**
**DE-A- 3 151 450**

**CHEMICAL ABSTRACTS, Band 108, 1988, Seite 254, Zusammenfassung Nr. 200229s, Columbus, Ohio, US; & JP-A-62 277 306 (KUMIAI CHEMICAL INDUSTRY CO., LTD) 02-12-1987**

(73) Patentinhaber: **HOECHST AKTIENGESELLSCHAFT**

**D-65926 Frankfurt(DE)**

(72) Erfinder: **Kehne, Heinz, Dr.**
**Berliner Strasse 10**
**D-6238 Hofheim am Taunus(DE)**
Erfinder: **Willms, Lothar, Dr.**
**Schulstrasse 3**
**D-5411 Hillscheid(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**
Erfinder: **Bürstell, Helmut, Dr.**
**Am Hohlacker 65**
**D-6000 Frankfurt am Main 50(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 342 569 B1

CHEMICAL ABSTRACTS, Band 108, 1988, Seite 249, Zusammenfassung Nr. 33622f, Columbus, Ohio, US; & JP-A-62 155 202 (KUMIAI CHEMICAL INDUSTRY CO., LTD) 10-07-1987

## Beschreibung

Es ist bekannt, daß heterocyclisch substituierte Phenoxysulfonylharnstoffe herbizide und pflanzenwachstumsregulierende Eigenschaften besitzen (EP-A 4163, DE-A 3151-450, Chem. Abstracts 108, 200229s = JP-A-62277306).
Überraschenderweise wurde nun gefunden, daß heterocyclisch substituierte Sulfamidsäurephenylester, deren Phenylesterteil von ausgewählten Brenzcatechinmonoalkylethern gebildet wird, als Herbizide oder Pflanzenwachstumsregulatoren besonders gut geeignet sind.

Gegenstand der vorliegenden Erfindung sind daher Verbindungen der Formel (I) oder deren Salze

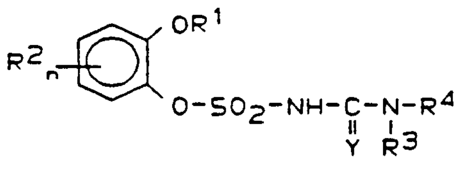

(I),

worin

| | |
|---|---|
| $R^1$ | Ethyl, Propyl oder Isopropyl, |
| $R^2$ | Halogen, $NO_2$, $CF_3$, CN, $(C_1-C_4)$Alkyl, $(C_1-C_4)$Alkoxy, $(C_1-C_4)$Alkylthio oder $(C_1-C_4$-Alkoxy)-carbonyl, |
| n | 0, 1, 2 oder 3, |
| Y | O oder S, |
| $R^3$ | Wasserstoff, $(C_1-C_8)$Alkyl, $(C_2-C_8)$Alkenyl, $(C_2-C_8)$Alkinyl oder $(C_1-C_4)$Alkoxy; |
| $R^4$ | einen heterocyclischen Rest der Formeln |

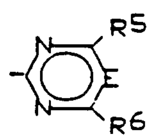 , 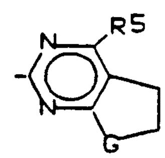 , 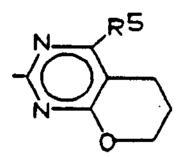 ,

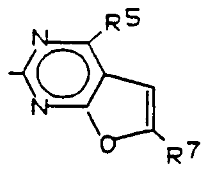 , 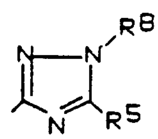 , 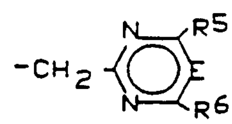 ,

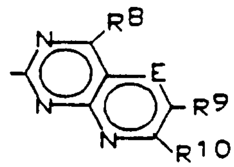 oder 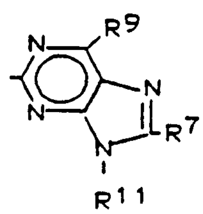

| | |
|---|---|
| E | CH oder N, |
| G | O oder $CH_2$, |
| $R^5$, $R^6$ | unabhängig voneinander Wasserstoff, $(C_1-C_6)$-Alkyl, $(C_1-C_6)$Alkoxy oder $(C_1-C_6)$Alkylthio, wobei die vorgenannten alkylhaltigen Reste ein- oder mehrfach durch Halogen oder ein- oder zweifach durch $(C_1-C_4)$Alkoxy oder $(C_1-C_4)$Alkylthio substituiert sein können, ferner einen Rest der Formel $-NR^{12}R^{13}$, $-OCHR^7-CO_2R^{12}$, $(C_3-C_6)$Cycloalkyl, $(C_3-C_5)$Alkenyl, $(C_2-C_4)$-Alkinyl, $(C_3-C_5)$- Alkenyloxy oder $(C_3-C_5)$Alkinyloxy, |
| $R^7$ | Wasserstoff oder $(C_1-C_4)$Alkyl, |

| | |
|---|---|
| $R^8$ | $(C_1-C_4)$Alkyl, $-CHF_2$ oder $-CH_2CF_3$, |
| $R^9$,$R^{10}$ | unabhängig voneinander Wasserstoff, $(C_1-C_4)$Alkyl, $(C_1-C_5)$Alkoxy oder Halogen, |
| $R^{11}$ | Wasserstoff, $(C_1-C_4)$Alkyl, $-CHF_2$ oder $CH_2CF_3$ und |
| $R^{12}$,$R^{13}$ | unabhängig voneinander Wasserstoff, $(C_1-C_4)$Alkyl, $(C_2-C_4)$Alkenyl oder $(C_3-C_4)$Alkinyl |

bedeuten.

Die Verbindungen der Formel (I) können Salze bilden, bei denen der Wasserstoff der $-SO_2$-NH-Gruppe durch ein für die Landwirtschaft geeignetes Kation ersetzt wird. Diese Salze sind im allgemeinen Metall-, insbesondere Alkali-, Erdalkali-, gegebenenfalls alkylierte Ammonium- oder organische Aminsalze.

Halogen bedeutet in obigen Definitionen vorzugsweise Fluor, Chlor oder Brom.

Bevorzugte Verbindungen der Formel (I) oder deren Salze sind solche, bei denen n = 0 oder 1, Y = 0, $R^2$ in Position 6 des Phenylrings orientiert ist und die obengenannte Bedeutung, im Falle Halogen insbesondere F oder Cl, besitzt, $R_3$ = Wasserstoff, $(C_1-C_4)$Alkyl oder $(C_3-C_4)$-Alkenyl, $R^4$ einen heterocylischen Rest der Formel

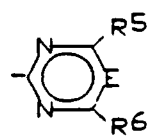 ,

E = CH oder N und $R^5$, $R^6$ $(C_1-C_4)$Alkyl, $(C_1-C_4)$Alkoxy oder $(C_1-C_4)$Alkylthio, die wie oben beschrieben substituiert sein können, bedeuten.

Besonders bevorzugte Verbindungen der Formel (I) oder deren Salze sind solche worin n = 0 oder 1, $R^2$ in Position 6 des Phenylringes orientiert ist und Fluor, Chlor, $(C_1-C_4)$Alkyl, $(C_1-C_4)$Alkoxy oder $(C_1-C_4)$-Alkoxycarbonyl bedeutet, $R^3$ = Wasserstoff oder Methyl, $R^4$ einen heterocyclischen Rest der Formel

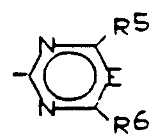

E = CH oder N und $R^5$ und $R^6$ unabhängig voneinander $(C_1-C_4)$-Alkyl, $(C_1-C_4)$Alkoxy, $OCHF_2$, $OCH_2CF_3$ oder $CF_3$, insbesondere (C1-C2)Alkyl oder (C1-C2)Alkoxy, bedeutet.

Weiterer Gegenstand der vorliegenden Erfindung sind Verfahren zur Herstellung der Verbindungen der allgemeinen Formel (I) oder deren Salze, dadurch gekennzeichnet, daß man

(a) eine Verbindung der Formel (II)

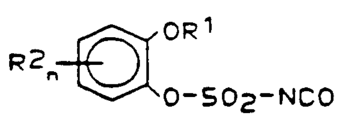 (II)

mit einer Verbindung der Formel (III)

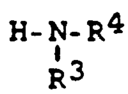 (III)

umsetzt, oder

(b) eine Verbindung der Formel (IV)

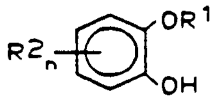

(IV)

mit einem Chlorsulfonylharnstoff der Formel (V)

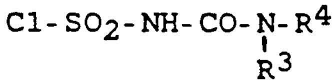

(V)

umsetzt, oder
(c) eine Verbindung der Formel (VI)

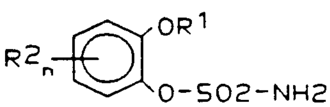

(VI)

mit einem Carbamat der Formel (VII)

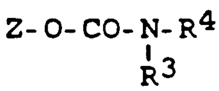

(VII)

worin Z Phenyl oder ($C_1$-$C_6$)Alkyl bedeutet, umsetzt, und die erhaltenen Verbindungen der Formel I gegebenenfalls in ihre Salze überführt.

Die Umsetzung der Verbindung der Formeln (II) und (III) erfolgt vorzugsweise in inerten aprotischen Lösungsmitteln, wie z.B. Acetonitril, Dichlormethan, Toluol, Tetrahydrofuran oder Dioxan bei Temperaturen zwischen 0 ° C und der Siedetermperatur des Lösungsmittels.

Die Phenoxysulfonylisocyanate der Formeln (II) lassen sich nach im Prinzip bekannten Verfahren aus den entsprechenden Brenzcatechinmonoethern der Formel (IV) und Chlorsulfonylisocyanat in einfacher Weise herstellen (vgl. G. Lohaus, Chem. Ber. 105, 2791 (1972)).

Die Ausgangsstoffe der Formel (III) sind bekannt oder lassen sich nach im Prinzip bekannten Verfahren herstellen, z.B. durch Cyclisierung entsprechender Guanidinderivate mit entsprechend substituierten 1,3-Diketonen, vergleiche z. B. "The Chemistry of Heterocyclic Compounds", Vol. XVI (1962) and Supplement I (1970), oder durch Derivatisierung von Cyanur-chlorid, vgl. z. B. "The Chemistry of Heterocyclic Compounds", L. Rapaport: "s-Triazines and Derivatives" (1959).

Die Umsetzung der Verbindung (IV) mit den Chlorsulfonylharnstoffen (V) führt man vorzugsweise in inerten Lösungsmitteln wie z. B. Dichlormethan bei Temperaturen zwischen -10 ° C und 80 ° C in Gegenwart einer Base als HC1-bindendes Agens durch. Als Basen können Alkali- oder Erdalkalicarbonate bzw. -bicarbonate wie z. B. $K_2CO3$, $NaHCO_3$, $Na_2CO_3$ oder tertiäre Amine wie z. B. Pyridin oder Triethylamin eingesetzt werden.

Die Brenzcatechinmonoether (IV) sind literaturbekannt oder können nach literaturbekannten Verfahren hergestellt werden. Die Chlorsulfonylharnstoffe (V) sind aus den Aminen der Formel (III) und Chlorsulfonylisocyanat zugänglich (EP-A 141 199).

Die Umsetzung der Verbindungen (VI) mit den heterocyclischen Carbamaten der Formel (VII) wird vorzugsweise in Gegenwart von tertiären organischen Basen wie z. B. 1,8-Diazabicyclo[5.4.0]-undec-7-en (DBU) in inerten Lösungsmitteln wie Acetonitril oder Dioxan bei Temperaturen zwischen 20 ° C und der Siedetemperatur des Lösungsmittels durchgeführt (analog EP-A 44 807).

Die hierzu erforderlichen Carbamate (VII) sind literaturbekannt oder werden nach bekannten Verfahren hergestellt (EP-A 70 804). Die Sulfamate (VI) werden nach bekannten Verfahren aus den zugrundeliegenden Brenzcatechinmonoethern hergestellt (vgl. z. B. Synthesis 1978, 357; Z. Chem. 15, 270 (1975); Chem. Ber. 105, 2791 (1972)).

Die Salze der Verbindungen der Formel I werden vorzugsweise in inerten Lösungsmitteln wie z. B. Wasser, Methanol oder Aceton bei Temperaturen von 0 - 100 °C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt.

Bei dikotylen Unkrautarten erstreckt sich das Wirkungsspektrum auf Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den perennierenden Unkräutern.

Unter den spezifischen Kulturbedingungen im Reis vorkommende Unkräuter wie z.B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, Emulsionen, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei

flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man auch Vermahlen der Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder auch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 10 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 EO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 EO) als Emulgator.

CHEMISCHE BEISPIELE

BEISPIEL 1

2-Ethoxyphenoxysulfonylisocyanat

Zu einer Lösung von 55,2 g (o,4 mol) 2-Ethoxyphenol in 500 ml Xylol tropft man bei 25 °C 67,9 g (0,48 mol) Chlorsulfonylisocyanat. Nach Beendigung des Zutropfens steigert man die Temperatur langsam auf 140 °C und erhitzt 2,5 h unter Rückfluß. Man kühlt ab und entfernt das Lösungsmittel, sowie überschüssiges Chlorsulfonylisocyanat am Rotationsverdampfer. Das zurückbleibende gelbe Öl (97,2 g = 100 % d.Th.)

wird ohne weitere Reinigung eingesetzt.

BEISPIEL 2

3-(4,6-Dimethoxypyrimidin-2-yl)-1-(2-ethoxyphenoxysulfonyl)-harnstoff

Zu 62,0 g (0,4 mol) 2-Amino-4,6-dimethoxypyrimidin in 600 ml Dichlormethan tropft man bei 25 °C eine Lösung aus 97,2 g (0,4 mol) des Produkts aus Beispiel 1 in 100 ml Dichlormethan. Man rührt 16 h bei Raumtemperatur nach, verdünnt mit 600 ml Dichlormethan und wäscht die organ. Phase 2 x mit je 500 ml 2N Salzsäure und 1 x mit 500 ml Wasser. Nach Trocknen mit Natriumsulfat und Entfernen des Lösungsmittel am Rotationsverdampfer hinterbleibt ein öliges Produkt, das beim Verreiben mit Diethylether kristallisiert. Man erhält 145,0 g (91 % d. Th.) 3-(4,6-Dimethoxypyrimidin-2-yl)-1-(2-ethoxyphenoxysulfonyl)-harnstoff vom Schmp. 145-147 °C.

BEISPIEL 3

3-(4,6-Dimethoxypyrimidin-2-yl)-1-(2-propoxyphenoxysulfonyl)-harnstoff

2,32 g (0,0084 mol) Phenyl-N-(4,6-dimethoxypyrimidin-2-yl)-carbamat werden in 100 ml Acetonitril gelöst und bei Raumtemperatur mit 1,74 g (0,008 mol) 2-Propoxyphenylsulfamat versetzt. Nach Zugabe von 1,33 g (0,0088 mol) 1,8-Diazabicyclo[5.4.0]undcec-7-en (DBU) wird das Reaktionsgemisch 18 h bei Raumtemperatur gerührt, eingeengt, mit $H_2O$ verdünnt und mit 2N Salzsäure auf pH 3-4 angesäuert. Nach Absaugen und Trocknen erhält man 2,85 g (86 % d. Th.) 3-(4,6-Dimethoxypyrimidin-2-yl)-1-(2-propoxyphenoxysulfonyl)-harnstoff vom Schmp. 108-109 °C.

Die Verbindungen der nachfolgenden Tabellen werden, wie in Beispiel 1-3 beschrieben, hergestellt.

**Tabelle 1**

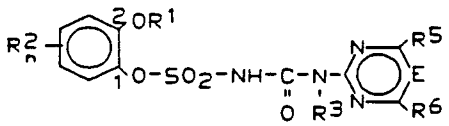

| Bsp.Nr. | $R^1$ | $R^3$ | $R^5$ | $R^6$ | E | n | Fp. [°C] |
|---|---|---|---|---|---|---|---|
| 4 | $CH_2CH_3$ | H | $CH_3$ | $CH_3$ | CH | 0 | 162 |
| 5 | $CH_2CH_3$ | H | $OCH_3$ | $CH_3$ | CH | 0 | 125 |
| 6 | $CH_2CH_3$ | H | $CH_3$ | $CH_3$ | N | 0 | |
| 7 | $CH_2CH_3$ | H | $OCH_3$ | $CH_3$ | N | 0 | 128-129 |
| 8 | $CH_2CH_3$ | H | $OCH_3$ | $OCH_3$ | N | 0 | 169-170 |
| 9 | $CH_2CH_3$ | H | $OCH_3$ | $SCH_3$ | N | 0 | |
| 10 | $CH_2CH_3$ | H | $OCF_2H$ | $CH_3$ | CH | 0 | |
| 11 | $CH_2CH_3$ | H | $OCF_2H$ | $OCF_2H$ | CH | 0 | 140 |
| 12 | $CH_2CH_3$ | H | $OCH_3$ | H | CH | 0 | |
| 13 | $CH_2CH_3$ | H | $OCH_3$ | $NHCH_3$ | CH | 0 | |
| 14 | $CH_2CH_3$ | H | $OCH_3$ | $NHCH_3$ | N | 0 | |
| 15 | $CH_2CH_3$ | H | $CH_3$ | $NHCH_3$ | CH | 0 | |
| 16 | $CH_2CH_3$ | H | $OC_2H_5$ | $NHCH_3$ | N | 0 | 168 |
| 17 | $CH_2CH_3$ | H | $OCH_3$ | $SCH_3$ | CH | 0 | |
| 18 | $CH_2CH_3$ | H | $OCH_3$ | $OC_2H_5$ | CH | 0 | |
| 19 | $CH_2CH_3$ | H | $OCH_3$ | $OC_3H_7$ | CH | 0 | |
| 20 | $CH_2CH_3$ | H | $OCH_3$ | $OC_2H_5$ | N | 0 | |
| 21 | $CH_2CH_3$ | H | $OC_2H_5$ | $OC_2H_5$ | CH | 0 | |
| 22 | $CH_2CH_3$ | H | $C_2H_5$ | $OCH_3$ | CH | 0 | |
| 23 | $CH_2CH_3$ | H | $CF_3$ | $OCH_3$ | CH | 0 | |
| 24 | $CH_2CH_3$ | H | $OCH_2CF_3$ | $CH_3$ | CH | 0 | |
| 25 | $CH_2CH_3$ | H | $OCH_2CF_3$ | $OCH_3$ | CH | 0 | |
| 26 | $CH_2CH_3$ | H | $OCH_2CF_3$ | $OCH_2CF_3$ | CH | 0 | |

| Bsp.Nr. | $R^1$ | $R^3$ | $R^5$ | $R^6$ | E | n | Fp. [°C] |
|---|---|---|---|---|---|---|---|
| 27 | $CH_2CH_3$ | H | $OCH_2CF_3$ | $NHCH_3$ | CH | 0 | |
| 28 | $CH_2CH_3$ | H | $OCH_2CF_3$ | $OCH_3$ | N | 0 | 175 |
| 29 | $CH_2CH_3$ | H | $OCH_2CF_3$ | $NHCH_3$ | N | 0 | |
| 30 | $CH_2CH_3$ | H | $OCH_3$ | $NHC_2H_5$ | CH | 0 | |
| 31 | $CH_2CH_3$ | H | $OCH_2CF_3$ | $NHC_2H_5$ | CH | 0 | |
| 32 | $CH_2CH_3$ | H | $OCH_3$ | $N(CH_3)_2$ | CH | 0 | |
| 33 | $CH_2CH_3$ | H | $OCH_3$ | $CH(OCH_3)_2$ | CH | 0 | |
| 34 | $CH_2CH_2CH_3$ | H | $CH_3$ | $CH_3$ | CH | 0 | 113-115 |
| 35 | $CH_2CH_2CH_3$ | H | $OCH_3$ | $CH_3$ | CH | 0 | 101-102 |
| 36 | $CH_2CH_2CH_3$ | H | $CH_3$ | $CH_3$ | N | 0 | |
| 37 | $CH_2CH_2CH_3$ | H | $OCH_3$ | $CH_3$ | N | 0 | 93-123 |
| 38 | $CH_2CH_2CH_3$ | H | $OCH_3$ | $OCH_3$ | N | 0 | |
| 39 | $CH_2CH_2CH_3$ | H | $OCF_2H$ | $CH_3$ | CH | 0 | 126 |
| 40 | $CH_2CH_2CH_3$ | H | $OCF_2H$ | $OCF_2H$ | CH | 0 | |
| 41 | $CH_2CH_2CH_3$ | H | $OCH_3$ | H | CH | 0 | |
| 42 | $CH_2CH_2CH_3$ | H | $OCH_3$ | $NHCH_3$ | CH | 0 | |
| 43 | $CH_2CH_2CH_3$ | H | $OCH_3$ | $NHCH_3$ | N | 0 | |
| 44 | $CH_2CH_2CH_3$ | H | CH3 | $NHCH_3$ | CH | 0 | |
| 45 | $CH_2CH_2CH_3$ | H | $CH_3$ | $NHCH_3$ | N | 0 | |
| 46 | $CH_2CH_2CH_3$ | H | $OCH_3$ | $SCH_3$ | CH | 0 | |
| 47 | $CH_2CH_2CH_3$ | H | $OCH_3$ | $OC_2H_5$ | CH | 0 | 127-130 |
| 48 | $CH_2CH_2CH_3$ | H | $OCH_3$ | $OC_3H_7$ | N | 0 | |
| 49 | $CH_2CH_2CH_3$ | H | $OCH_3$ | $OC_2H_5$ | N | 0 | |
| 50 | $CH_2CH_2CH_3$ | H | $OC_2H_5$ | $OC_2H_5$ | CH | 0 | |
| 51 | $CH_2CH_2CH_3$ | H | $C_2H_5$ | $OCH_3$ | CH | 0 | |
| 52 | $CH_2CH_2CH_3$ | H | $CF_3$ | $OCH_3$ | CH | 0 | |
| 53 | $CH_2CH_2CH_3$ | H | $OCH_2CF_3$ | $CH_3$ | CH | 0 | |
| 54 | $CH_2CH_2CH_3$ | H | $OCH_2CF_3$ | $OCH_3$ | CH | 0 | |
| 55 | $CH_2CH_2CH_3$ | H | $OCH_2CF_3$ | $OCH_2CF_3$ | CH | 0 | |

| Bsp.Nr. | $R^1$ | $R^3$ | $R^5$ | $R^6$ | E | n | Fp. [°C] |
|---|---|---|---|---|---|---|---|
| 56 | $CH_2CH_2CH_3$ | H | $OCH_2CF_3$ | $NHCH_3$ | CH | 0 | |
| 57 | $CH_2CH_2CH_3$ | H | $OCH_2CF_3$ | $OCH_3$ | N | 0 | |
| 58 | $CH_2CH_2CH_3$ | H | $OCH_2CF_3$ | $NHCH_3$ | N | 0 | |
| 59 | $CH_2CH_2CH_3$ | H | $OCH_3$ | $OCH_3$ | CH | 0 | |
| 60 | $CH_2CH_2CH_3$ | H | $OCH_2CF_3$ | $NHC_2H_5$ | CH | 0 | |
| 61 | $CH_2CH_2CH_3$ | H | $OCH_3$ | $N(CH_3)_2$ | CH | 0 | |
| 62 | $CH_2CH_2CH_3$ | H | $OCH_3$ | $CH(OCH_3)_2$ | CH | 0 | |
| 63 | $CH(CH_3)_2$ | H | $CH_3$ | $CH_3$ | CH | 0 | 90-92 |
| 64 | $CH(CH_3)_2$ | H | $OCH_3$ | $CH_3$ | CH | 0 | 135-137 |
| 65 | $CH(CH_3)_2$ | H | $CH_3$ | $CH_3$ | N | 0 | |
| 66 | $CH(CH_3)_2$ | H | $OCH_3$ | $CH_3$ | N | 0 | 108-110 |
| 67 | $CH(CH_3)_2$ | H | $OCH_3$ | $OCH_3$ | CH | 0 | 141-143 |
| 68 | $CH(CH_3)_2$ | H | $OCF_2H$ | $CH_3$ | CH | 0 | |
| 69 | $CH(CH_3)_2$ | H | $OCF_2H$ | $OCF_2H$ | CH | 0 | 135-136 |
| 70 | $CH(CH_3)_2$ | H | $OCH_3$ | H | CH | 0 | |
| 71 | $CH(CH_3)_2$ | H | $OCH_3$ | $NHCH_3$ | CH | 0 | |
| 72 | $CH(CH_3)_2$ | H | $OCH_3$ | $NHCH_3$ | N | 0 | |
| 73 | $CH(CH_3)_2$ | H | $CH_3$ | $NHCH_3$ | CH | 0 | |
| 74 | $CH(CH_3)_2$ | H | $CH_3$ | $NHCH_3$ | N | 0 | |
| 75 | $CH(CH_3)_2$ | H | $OCH_3$ | $SCH_3$ | CH | 0 | |
| 76 | $CH(CH_3)_2$ | H | $OCH_3$ | $OC_2H_5$ | CH | 0 | |
| 77 | $CH(CH_3)_2$ | H | $OCH_3$ | $OC_3H_7$ | CH | 0 | |
| 78 | $CH(CH_3)_2$ | H | $OCH_3$ | $OC_2H_5$ | N | 0 | |
| 79 | $CH(CH_3)_2$ | H | $OC_2H_5$ | $OC_2H_5$ | CH | 0 | |
| 80 | $CH(CH_3)_2$ | H | $C_2H_5$ | $OCH_3$ | CH | 0 | |
| 81 | $CH(CH_3)_2$ | H | $CF_3$ | $OCH_3$ | CH | 0 | |
| 82 | $CH(CH_3)_2$ | H | $OCH_2CF_3$ | $CH_3$ | CH | 0 | |
| 83 | $CH(CH_3)_2$ | H | $OCH_2CF_3$ | $OCH_3$ | CH | 0 | |
| 84 | $CH(CH_3)_2$ | H | $OCH_2CF_3$ | $OCH_2CF_3$ | CH | 0 | |

| Bsp.Nr. | $R^1$ | $R^2$ | $R^3$ | $R^5$ | $R^6$ | E | n | Fp. |
|---|---|---|---|---|---|---|---|---|
| 85 | $CH(CH_3)_2$ | | H | $OCH_3$ | $OCH_3$ | N | 0 | |
| 86 | $CH(CH_3)_2$ | | H | $OCH_2CF_3$ | $OCH_3$ | N | 0 | |
| 87 | $CH(CH_3)_2$ | | H | $OCH_2CF_3$ | $NHCH_3$ | N | 0 | |
| 88 | $CH(CH_3)_2$ | | H | $OCH_3$ | $NHC_2H_5$ | CH | 0 | |
| 89 | $CH(CH_3)_2$ | | H | $OCH_2CF_3$ | $NHC_2H_5$ | CH | 0 | |
| 90 | $CH(CH_3)_2$ | | H | $OCH_3$ | $N(CH_3)_2$ | CH | 0 | |
| 91 | $CH(CH_3)_2$ | | H | $OCH_3$ | $CH(OCH_3)_2$ | CH | 0 | |
| 92 | $CH_2CH_3$ | 6-Cl | H | $OCH_3$ | $OCH_3$ | CH | 1 | 162-163 |
| 93 | $CH_2CH_3$ | 6-Cl | H | $OCH_3$ | $CH_3$ | CH | 1 | 151-152 |
| 94 | $CH_2CH_3$ | 6-Cl | H | $OCH_3$ | $CH_3$ | N | 1 | 128-129 |
| 95 | $CH_2CH_3$ | 6-F | H | $OCH_3$ | $OCH_3$ | CH | 1 | |
| 96 | $CH_2CH_3$ | 6-F | H | $OCH_3$ | $CH_3$ | CH | 1 | |
| 97 | $CH_2CH_3$ | 6-F | H | $OCH_3$ | $CH_3$ | N | 1 | |
| 98 | $CH_2CH_3$ | 6-Br | H | $OCH_3$ | $OCH_3$ | CH | 1 | |
| 99 | $CH_2CH_3$ | 5-F | H | $OCH_3$ | $CH_3$ | CH | 1 | |
| 100 | $CH_2CH_3$ | 5-Br | H | $OCH_3$ | $CH_3$ | N | 1 | |
| 101 | $CH_2CH_3$ | 5-Cl | H | $OCH_3$ | $OCH_3$ | CH | 1 | |
| 102 | $CH_2CH_3$ | 4-Cl | H | $OCH_3$ | $CH_3$ | CH | 1 | |
| 103 | $CH_2CH_3$ | 4-F | H | $OCH_3$ | $CH_3$ | N | 1 | |
| 104 | $CH_2CH_3$ | 3-Cl | H | $OCH_3$ | $OCH_3$ | CH | 1 | 148-149 |
| 105 | $CH_2CH_3$ | 3-F | H | $OCH_3$ | $CH_3$ | CH | 1 | |
| 106 | $CH_2CH_3$ | 6-$OCH_3$ | H | $OCH_3$ | $CH_3$ | N | 1 | |
| 107 | $CH_2CH_3$ | 6-$OCH_3$ | H | $OCH_3$ | $OCH_3$ | CH | 1 | |
| 108 | $CH_2CH_3$ | 6-$OCH_3$ | H | $OCH_3$ | $CH_3$ | CH | 1 | |
| 109 | $CH_2CH_3$ | 5-$OCH_3$ | H | $OCH_3$ | $CH_3$ | N | 1 | |
| 110 | $CH_2CH_3$ | 4-$OCH_3$ | H | $OCH_3$ | $OCH_3$ | CH | 1 | |
| 112 | $CH_2CH_3$ | 4-$OCH_3$ | H | $OCH_3$ | $CH_3$ | CH | 1 | |
| 113 | $CH_2CH_3$ | 3-$OCH_3$ | H | $OCH_3$ | $CH_3$ | N | 1 | |

| Bsp.Nr. | $R^1$ | $R^2$ | $R^3$ | $R^5$ | $R^6$ | E | n | Fp. |
|---|---|---|---|---|---|---|---|---|
| 114 | $CH_2CH_3$ | 6-$OC_2H_5$ | H | $OCH_3$ | $OCH_3$ | CH | 1 | 194-195 |
| 115 | $CH_2CH_3$ | 6-$OC_3H_7$ | H | $OCH_3$ | $CH_3$ | CH | 1 | |
| 116 | $CH_2CH_3$ | 6-$OC_4H_9$ | H | $OCH_3$ | $CH_3$ | N | 1 | |
| 117 | $CH_2CH_3$ | 6-$COOCH_3$ | H | $OCH_3$ | $OCH_3$ | CH | 1 | 163 |
| 118 | $CH_2CH_3$ | 6-$COOCH_3$ | H | $OCH_3$ | $CH_3$ | CH | 1 | 148 |
| 119 | $CH_2CH_3$ | 6-$COOCH_3$ | H | $OCH_3$ | $CH_3$ | N | 1 | |
| 120 | $CH_2CH_3$ | 6-$COOC_2H_5$ | H | $OCH_3$ | $OCH_3$ | CH | 1 | 124-125 |
| 121 | $CH_2CH_3$ | 6-$COOC_2H_5$ | H | $OCH_3$ | $CH_3$ | CH | 1 | 128-129 |
| 122 | $CH_2CH_3$ | 5-$COOCH_3$ | H | $OCH_3$ | $CH_3$ | N | 1 | |
| 123 | $CH_2CH_3$ | 4-$COOCH_3$ | H | $OCH_3$ | $OCH_3$ | CH | 1 | |
| 124 | $CH_2CH_3$ | 4-$COOCH_3$ | H | $OCH_3$ | $CH_3$ | CH | 1 | |
| 125 | $CH_2CH_3$ | 3-$COOCH_3$ | H | $OCH_3$ | $CH_3$ | N | 1 | |
| 126 | $CH_2CH_3$ | 6-$COOC_3H_7$ | H | $OCH_3$ | $OCH_3$ | CH | 1 | |
| 127 | $CH_2CH_3$ | 6-$COOC_4H_9$ | H | $OCH_3$ | $CH_3$ | CH | 1 | |
| 128 | $CH_2CH_3$ | 6-$CH_3$ | H | $OCH_3$ | $CH_3$ | N | 1 | 139-140 |
| 129 | $CH_2CH_3$ | 6-$CH_3$ | H | $OCH_3$ | $OCH_3$ | CH | 1 | 177-178 |
| 130 | $CH_2CH_3$ | 6-$CH_3$ | H | $OCH_3$ | $CH_3$ | CH | 1 | 155-156(Zers.) |
| 131 | $CH_2CH_3$ | 5-$CH_3$ | H | $OCH_3$ | $CH_3$ | N | 1 | |
| 132 | $CH_2CH_3$ | 4-$CH_3$ | H | $OCH_3$ | $OCH_3$ | CH | 1 | |
| 133 | $CH_2CH_3$ | 3-$CH_3$ | H | $OCH_3$ | $CH_3$ | CH | 1 | 118-120 |
| 134 | $CH_2CH_3$ | 6-$C_2H_5$ | H | $OCH_3$ | $CH_3$ | N | 1 | 139-141 |
| 135 | $CH_2CH_3$ | 6-$C_4H_9$ | H | $OCH_3$ | $OCH_3$ | CH | 1 | |
| 136 | $CH_2CH_3$ | 6-$NO_2$ | H | $OCH_3$ | $CH_3$ | CH | 1 | |
| 137 | $CH_2CH_3$ | 6-$CF_3$ | H | $OCH_3$ | $CH_3$ | N | 1 | 155-156 |
| 138 | $CH_2CH_3$ | 6-$SCH_3$ | H | $OCH_3$ | $OCH_3$ | CH | 1 | |
| 139 | $CH_2CH_3$ | 6-$SC_2H_5$ | H | $OCH_3$ | $CH_3$ | CH | 1 | |
| 140 | $CH_2CH_3$ | 6-$SC_5H_9$ | H | $OCH_3$ | $CH_3$ | N | 1 | |

| Bsp.Nr. | $R^1$ | $R^2$ | $R^3$ | $R^5$ | $R^6$ | E | n | Fp. |
|---|---|---|---|---|---|---|---|---|
| 141 | $CH_2CH_2CH_3$ | 6-Cl | H | $OCH_3$ | $OCH_3$ | CH | 1 | |
| 142 | $CH_2CH_2CH_3$ | 6-Cl | H | $OCH_3$ | $CH_3$ | CH | 1 | |
| 143 | $CH_2CH_2CH_3$ | 6-Cl | H | $OCH_3$ | $CH_3$ | N | 1 | |
| 144 | $CH_2CH_2CH_3$ | 6-F | H | $OCH_3$ | $OCH_3$ | CH | 1 | |
| 145 | $CH_2CH_2CH_3$ | 6-F | H | $OCH_3$ | $CH_3$ | CH | 1 | |
| 146 | $CH_2CH_2CH_3$ | 6-F | H | $OCH_3$ | $CH_3$ | N | 1 | |
| 147 | $CH_2CH_2CH_3$ | 6-Br | H | $OCH_3$ | $OCH_3$ | CH | 1 | |
| 148 | $CH_2CH_2CH_3$ | 5-F | H | $OCH_3$ | $CH_3$ | CH | 1 | |
| 149 | $CH_2CH_2CH_3$ | 5-Br | H | $OCH_3$ | $CH_3$ | N | 1 | |
| 150 | $CH_2CH_2CH_3$ | 5-Cl | H | $OCH_3$ | $OCH_3$ | CH | 1 | |
| 151 | $CH_2CH_2CH_3$ | 4-Cl | H | $OCH_3$ | $CH_3$ | CH | 1 | |
| 152 | $CH_2CH_2CH_3$ | 4-F | H | $OCH_3$ | $CH_3$ | N | 1 | |
| 153 | $CH_2CH_2CH_3$ | 3-Cl | H | $OCH_3$ | $OCH_3$ | CH | 1 | |
| 154 | $CH_2CH_2CH_3$ | 3-F | H | $OCH_3$ | $CH_3$ | CH | 1 | |
| 155 | $CH_2CH_2CH_3$ | 6-$OCH_3$ | H | $OCH_3$ | $CH_3$ | N | 1 | |
| 156 | $CH_2CH_2CH_3$ | 6-$OCH_3$ | H | $OCH_3$ | $OCH_3$ | CH | 1 | |
| 157 | $CH_2CH_2CH_3$ | 6-$OCH_3$ | H | $OCH_3$ | $CH_3$ | CH | 1 | |
| 158 | $CH_2CH_2CH_3$ | 5-$OCH_3$ | H | $OCH_3$ | $CH_3$ | N | 1 | |
| 159 | $CH_2CH_2CH_3$ | 4-$OCH_3$ | H | $OCH_3$ | $OCH_3$ | CH | 1 | |
| 160 | $CH_2CH_2CH_3$ | 4-$OCH_3$ | H | $OCH_3$ | $CH_3$ | CH | 1 | |
| 161 | $CH_2CH_2CH_3$ | 3-$OCH_3$ | H | $OCH_3$ | $CH_3$ | N | 1 | |
| 162 | $CH_2CH_2CH_3$ | 6-$OC_2H_5$ | H | $OCH_3$ | $OCH_3$ | CH | 1 | |
| 163 | $CH_2CH_2CH_3$ | 6-$OC_3H_7$ | H | $OCH_3$ | $CH_3$ | CH | 1 | |
| 164 | $CH_2CH_2CH_3$ | 6-$OC_4H_9$ | H | $OCH_3$ | $CH_3$ | N | 1 | |

| Bsp.Nr. | $R^1$ | $R^2$ | $R^3$ | $R^5$ | $R^6$ | E | n | Fp. |
|---|---|---|---|---|---|---|---|---|
| 165 | $CH_2CH_2CH_3$ | 6-$COOCH_3$ | H | $OCH_3$ | $OCH_3$ | CH | 1 | |
| 166 | $CH_2CH_2CH_3$ | 6-$COOCH_3$ | H | $OCH_3$ | $CH_3$ | CH | 1 | |
| 167 | $CH_2CH_2CH_3$ | 6-$COOCH_3$ | H | $OCH_3$ | $CH_3$ | N | 1 | |
| 168 | $CH_2CH_2CH_3$ | 6-$COOC_2H_5$ | H | $OCH_3$ | $OCH_3$ | CH | 1 | |
| 169 | $CH_2CH_2CH_3$ | 6-$COOC_2H_5$ | H | $OCH_3$ | $CH_3$ | CH | 1 | |
| 170 | $CH_2CH_2CH_3$ | 5-$COOCH_3$ | H | $OCH_3$ | $CH_3$ | N | 1 | |
| 171 | $CH_2CH_2CH_3$ | 4-$COOCH_3$ | H | $OCH_3$ | $OCH_3$ | CH | 1 | |
| 172 | $CH_2CH_2CH_3$ | 4-$COOCH_3$ | H | $OCH_3$ | $CH_3$ | CH | 1 | |
| 173 | $CH_2CH_2CH_3$ | 3-$COOCH_3$ | H | $OCH_3$ | $CH_3$ | N | 1 | |
| 174 | $CH_2CH_2CH_3$ | 6-$COOC_3H_7$ | H | $OCH_3$ | $OCH_3$ | CH | 1 | |
| 175 | $CH_2CH_2CH_3$ | 6-$COOC_4H_9$ | H | $OCH_3$ | $CH_3$ | CH | 1 | |
| 176 | $CH_2CH_2CH_3$ | 6-$CH_3$ | H | $OCH_3$ | $CH_3$ | N | 1 | |
| 177 | $CH_2CH_2CH_3$ | 6-$CH_3$ | H | $OCH_3$ | $OCH_3$ | CH | 1 | |
| 178 | $CH_2CH_2CH_3$ | 6-$CH_3$ | H | $OCH_3$ | $CH_3$ | CH | 1 | |
| 179 | $CH_2CH_2CH_3$ | 5-$CH_3$ | H | $OCH_3$ | $CH_3$ | N | 1 | |
| 180 | $CH_2CH_2CH_3$ | 4-$CH_3$ | H | $OCH_3$ | $OCH_3$ | CH | 1 | |
| 181 | $CH_2CH_2CH_3$ | 3-$CH_3$ | H | $OCH_3$ | $CH_3$ | CH | 1 | |
| 182 | $CH_2CH_2CH_3$ | 6-$C_2H_5$ | H | $OCH_3$ | $CH_3$ | N | 1 | |
| 183 | $CH_2CH_2CH_3$ | 6-$C_4H_9$ | H | $OCH_3$ | $OCH_3$ | CH | 1 | |
| 184 | $CH_2CH_2CH_3$ | 6-$NO_2$ | H | $OCH_3$ | $CH_3$ | CH | 1 | |
| 185 | $CH_2CH_2CH_3$ | 6-$CF_3$ | H | $OCH_3$ | $CH_3$ | N | 1 | |
| 186 | $CH_2CH_2CH_3$ | 6-$SCH_3$ | H | $OCH_3$ | $OCH_3$ | CH | 1 | |
| 187 | $CH_2CH_2CH_3$ | 6-$SC_2H_5$ | H | $OCH_3$ | $CH_3$ | CH | 1 | |
| 188 | $CH_2CH_2CH_3$ | 6-$SC_5H_9$ | H | $OCH_3$ | $CH_3$ | N | 1 | |

| Bsp.Nr. | $R^1$ | $R^2$ | $R^3$ | $R^5$ | $R^6$ | E | n | Fp. |
|---|---|---|---|---|---|---|---|---|
| 189 | $CH(CH_3)_2$ | 6-Cl | H | $OCH_3$ | $OCH_3$ | CH | 1 | |
| 190 | $CH(CH_3)_2$ | 6-Cl | H | $OCH_3$ | $CH_3$ | CH | 1 | |
| 191 | $CH(CH_3)_2$ | 6-Cl | H | $OCH_3$ | $CH_3$ | N | 1 | |
| 192 | $CH(CH_3)_2$ | 6-F | H | $OCH_3$ | $OCH_3$ | CH | 1 | |
| 193 | $CH(CH_3)_2$ | 6-F | H | $OCH_3$ | $CH_3$ | CH | 1 | |
| 194 | $CH(CH_3)_2$ | 6-F | H | $OCH_3$ | $CH_3$ | N | 1 | |
| 195 | $CH(CH_3)_2$ | 6-Br | H | $OCH_3$ | $OCH_3$ | CH | 1 | |
| 196 | $CH(CH_3)_2$ | 5-F | H | $OCH_3$ | $CH_3$ | CH | 1 | |
| 197 | $CH(CH_3)_2$ | 5-Br | H | $OCH_3$ | $CH_3$ | N | 1 | |
| 198 | $CH(CH_3)_2$ | 5-Cl | H | $OCH_3$ | $OCH_3$ | CH | 1 | |
| 199 | $CH(CH_3)_2$ | 4-Cl | H | $OCH_3$ | $CH_3$ | CH | 1 | |
| 200 | $CH(CH_3)_2$ | 4-F | H | $OCH_3$ | $CH_3$ | N | 1 | |
| 201 | $CH(CH_3)_2$ | 3-Cl | H | $OCH_3$ | $OCH_3$ | CH | 1 | |
| 202 | $CH(CH_3)_2$ | 3-F | H | $OCH_3$ | $CH_3$ | CH | 1 | |
| 203 | $CH(CH_3)_2$ | 6-$OCH_3$ | H | $OCH_3$ | $CH_3$ | N | 1 | |
| 204 | $CH(CH_3)_2$ | 6-$OCH_3$ | H | $OCH_3$ | $OCH_3$ | CH | 1 | |
| 205 | $CH(CH_3)_2$ | 6-$OCH_3$ | H | $OCH_3$ | $CH_3$ | CH | 1 | |
| 206 | $CH(CH_3)_2$ | 5-$OCH_3$ | H | $OCH_3$ | $CH_3$ | N | 1 | |
| 207 | $CH(CH_3)_2$ | 4-$OCH_3$ | H | $OCH_3$ | $OCH_3$ | CH | 1 | |
| 208 | $CH(CH_3)_2$ | 4-$OCH_3$ | H | $OCH_3$ | $CH_3$ | CH | 1 | |
| 209 | $CH(CH_3)_2$ | 3-$OCH_3$ | H | $OCH_3$ | $CH_3$ | N | 1 | |
| 210 | $CH(CH_3)_2$ | 6-$OC_2H_5$ | H | $OCH_3$ | $OCH_3$ | CH | 1 | |
| 211 | $CH(CH_3)_2$ | 6-$OC_3H_7$ | H | $OCH_3$ | $CH_3$ | CH | 1 | |
| 212 | $CH(CH_3)_2$ | 6-$OC_4H_9$ | H | $OCH_3$ | $CH_3$ | N | 1 | |

| Bsp.Nr. | $R^1$ | $R^2$ | $R^3$ | $R^5$ | $R^6$ | E | n | Fp. |
|---|---|---|---|---|---|---|---|---|
| 213 | $CH(CH_3)_2$ | 6-$COOCH_3$ | H | $OCH_3$ | $OCH_3$ | CH | 1 | |
| 214 | $CH(CH_3)_2$ | 6-$COOCH_3$ | H | $OCH_3$ | $CH_3$ | CH | 1 | |
| 215 | $CH(CH_3)_2$ | 6-$COOCH_3$ | H | $OCH_3$ | $CH_3$ | N | 1 | |
| 216 | $CH(CH_3)_2$ | 6-$COOC_2H_5$ | H | $OCH_3$ | $OCH_3$ | CH | 1 | |
| 217 | $CH(CH_3)_2$ | 6-$COOC_2H_5$ | H | $OCH_3$ | $CH_3$ | CH | 1 | |
| 218 | $CH(CH_3)_2$ | 5-$COOCH_3$ | H | $OCH_3$ | $CH_3$ | N | 1 | |
| 219 | $CH(CH_3)_2$ | 4-$COOCH_3$ | H | $OCH_3$ | $OCH_3$ | CH | 1 | |
| 220 | $CH(CH_3)_2$ | 4-$COOCH_3$ | H | $OCH_3$ | $CH_3$ | CH | 1 | |
| 221 | $CH(CH_3)_2$ | 3-$COOCH_3$ | H | $OCH_3$ | $CH_3$ | N | 1 | |
| 222 | $CH(CH_3)_2$ | 6-$COOC_3H_7$ | H | $OCH_3$ | $OCH_3$ | CH | 1 | |
| 223 | $CH(CH_3)_2$ | 6-$COOC_4H_9$ | H | $OCH_3$ | $CH_3$ | CH | 1 | |
| 224 | $CH(CH_3)_2$ | 6-$CH_3$ | H | $OCH_3$ | $CH_3$ | N | 1 | |
| 225 | $CH(CH_3)_2$ | 6-$CH_3$ | H | $OCH_3$ | $OCH_3$ | CH | 1 | |
| 226 | $CH(CH_3)_2$ | 6-$CH_3$ | H | $OCH_3$ | $CH_3$ | CH | 1 | |
| 227 | $CH(CH_3)_2$ | 5-$CH_3$ | H | $OCH_3$ | $CH_3$ | N | 1 | |
| 228 | $CH(CH_3)_2$ | 4-$CH_3$ | H | $OCH_3$ | $OCH_3$ | CH | 1 | |
| 229 | $CH(CH_3)_2$ | 3-$CH_3$ | H | $OCH_3$ | $CH_3$ | CH | 1 | |
| 230 | $CH(CH_3)_2$ | 6-$C_2H_5$ | H | $OCH_3$ | $CH_3$ | N | 1 | |
| 231 | $CH(CH_3)_2$ | 6-$C_4H_9$ | H | $OCH_3$ | $OCH_3$ | CH | 1 | |
| 232 | $CH(CH_3)_2$ | 6-$NO_2$ | H | $OCH_3$ | $CH_3$ | CH | 1 | |
| 233 | $CH(CH_3)_2$ | 6-$CF_3$ | H | $OCH_3$ | $CH_3$ | N | 1 | |
| 234 | $CH(CH_3)_2$ | 6-$SCH_3$ | H | $OCH_3$ | $OCH_3$ | CH | 1 | |
| 235 | $CH(CH_3)_2$ | 6-$SC_2H_5$ | H | $OCH_3$ | $CH_3$ | CH | 1 | |
| 236 | $CH(CH_3)_2$ | 6-$SC_5H_9$ | H | $OCH_3$ | $CH_3$ | N | 1 | |

| Bsp.Nr. | $R^1$ | $R^2$ | $R^3$ | $R^5$ | $R^6$ | E | n | Fp. |
|---|---|---|---|---|---|---|---|---|
| 237 | $CH_2CH_3$ | 4,6-$Cl_2$ | H | $OCH_3$ | $OCH_3$ | CH | 2 | |
| 238 | $CH_2CH_3$ | 4,6-$Cl_2$ | H | $OCH_3$ | $CH_3$ | N | 2 | |
| 239 | $CH_2CH_3$ | 3,5-$Cl_2$ | H | $OCH_3$ | $OCH_3$ | CH | 2 | |
| 240 | $CH_2CH_2CH_3$ | 4,6-$Cl_2$ | H | $OCH_3$ | $OCH_3$ | CH | 2 | |
| 241 | $CH_2CH_2CH_3$ | 4,6-$Cl_2$ | H | $OCH_3$ | $CH_3$ | CH | 2 | |
| 242 | $CH_2CH_2CH_3$ | 3,5-$Cl_2$ | H | $OCH_3$ | $OCH_3$ | CH | 2 | |
| 243 | $CH(CH_3)_2$ | 4,6-$Cl_2$ | H | $OCH_3$ | $CH_3$ | N | 2 | |
| 244 | $CH(CH_3)_2$ | 4,6-$Cl_2$ | H | $OCH_3$ | $OCH_3$ | CH | 2 | |
| 245 | $CH(CH_3)_2$ | 3,5-$Cl_2$ | H | $OCH_3$ | $OCH_3$ | CH | 2 | |
| 246 | $CH_2CH_3$ | 4,6-$F_2$ | H | $OCF_2H$ | $CH_3$ | CH | 2 | |
| 247 | $CH_2CH_3$ | 4,6-$F_2$ | H | $OCH_3$ | $OCH_3$ | CH | 2 | |
| 248 | $CH_2CH_3$ | 4,6-$F_2$ | H | $OCH_3$ | $CH_3$ | N | 2 | |
| 249 | $CH_2CH_3$ | 3,5-$F_2$ | H | $OCH_3$ | $OCH_3$ | CH | 2 | |
| 250 | $CH_2CH_2CH_3$ | 4,6-$F_2$ | H | $OCH_3$ | $OCH_3$ | CH | 2 | |
| 251 | $CH_2CH_2CH_3$ | 4,6-$F_2$ | H | $OCF_2H$ | $OCF_2H$ | CH | 2 | |
| 252 | $CH_2CH_2CH_3$ | 3,5-$F_2$ | H | $OCH_3$ | $OCH_3$ | CH | 2 | |
| 253 | $CH(CH_3)_2$ | 4,6-$F_2$ | H | $OCH_3$ | $CH_3$ | N | 2 | |
| 254 | $CH(CH_3)_2$ | 4,6-$F_2$ | H | $OCH_3$ | $OCH_3$ | CH | 2 | |
| 255 | $CH_2CH_3$ | 4,6-$(CH_3)_2$ | H | $OCH_3$ | $OCH_3$ | CH | 2 | |
| 256 | $CH_2CH_3$ | 3,5-$(NO_2)_2$ | H | $OCH_2CF_3$ | $OCH_3$ | CH | 2 | |
| 257 | $CH_2CH_3$ | H | $CH_3$ | $OCH_3$ | $OCH_3$ | CH | 0 | 119-120 |
| 258 | $CH_2CH_3$ | H | $CH_3$ | $OCH_3$ | $CH_3$ | N | 0 | 101-102 |
| 259 | $CH_2CH_3$ | H | $CH_2CH{=}CH_2$ | $OCH_3$ | $OCH_3$ | CH | 0 | |
| 260 | $CH_2CH_2CH_3$ | H | $CH_3$ | $OCH_3$ | $OCH_3$ | CH | 0 | |
| 261 | $CH_2CH_2CH_3$ | H | $CH_3$ | $OCH_3$ | $CH_3$ | CH | 0 | |
| 262 | $CH_2CH_2CH_3$ | H | $CH_2CH{=}CH_2$ | $OCH_3$ | $OCH_3$ | CH | 0 | |
| 263 | $CH_2CH_2CH_3$ | H | $CH_2CH{=}CH_2$ | $OCH_3$ | $CH_3$ | N | 0 | |

| Bsp.Nr. | $R^1$ | $R^2$ | $R^3$ | $R^5$ | $R^6$ | E | n | Fp. |
|---|---|---|---|---|---|---|---|---|
| 264 | $CH(CH_3)_2$ | H | $CH_3$ | $OCH_3$ | $OCH_3$ | CH | 0 | 127-130 |
| 265 | $CH(CH_3)_2$ | H | $CH_2CH{=}CH_2$ | $OCH_3$ | $OCH_3$ | CH | 0 | |
| 266 | $CH(CH_3)_2$ | H | $CH_2CH{=}CH_2$ | $OCF_2H$ | $CH_3$ | CH | 0 | |
| 267 | $CH_2CH_3$ | H | $C_2H_5$ | $OCH_3$ | $OCH_3$ | CH | 0 | |
| 268 | $CH(CH_3)_2$ | H | $C_2H_5$ | $OCH_3$ | $OCH_3$ | CH | 0 | |
| 269 | $CH_2CH_3$ | H | $C_2H_5$ | $OCH_2CF_3$ | $OCH_3$ | CH | 0 | |
| 270 | $CH_2CH_2CH_3$ | H | $C_2H_5$ | $OCH_3$ | $OCH_3$ | CH | 0 | |
| 271 | $CH_2CH_3$ | 6-$CH_3$ | $CH_3$ | $OCH_3$ | $CH_3$ | N | 1 | |
| 272 | $CH_2CH_2CH_3$ | 6-$OCH_3$ | $CH_3$ | $OCH_3$ | $OCH_3$ | CH | 1 | |
| 273 | $CH_2CH_3$ | 6-F | $CH_2CH{=}CH_2$ | $OCH_3$ | $OCH_3$ | CH | 1 | |
| 274 | $CH_2CH_2CH_3$ | 6-$CF_3$ | $CH_3$ | $OCH_2CF_3$ | $OCH_3$ | N | 1 | |
| 275 | $CH(CH_3)_2$ | 6-$COOCH_3$ | $CH_3$ | $OCH_3$ | $OCH_3$ | CH | 1 | |
| 276 | $CH_2CH_3$ | 6-$C_2H_5$ | H | $OCH_3$ | $OCH_3$ | CH | 1 | 166-167 |
| 277 | " | " | H | $OCH_3$ | $CH_3$ | CH | 1 | 155-156 |
| 278 | " | " | H | $CH_3$ | $CH_3$ | CH | 1 | |
| 279 | " | " | H | $OCH_3$ | $OCH_2CF_3$ | N | 1 | |
| 280 | " | " | $CH_3$ | $OCH_3$ | $OCH_3$ | CH | 1 | |
| 281 | " | " | $CH_3$ | $OCH_3$ | $CH_3$ | N | 1 | |
| 282 | " | " | H | $OCF_2H$ | $OCF_2H$ | CH | 1 | |
| 283 | " | " | H | $OCH_3$ | $OCH_3$ | N | 1 | |
| 284 | " | 6-$C_3H_7$ | H | $OCH_3$ | $OCH_3$ | CH | 1 | 158 |
| 285 | " | " | H | $OCH_3$ | $CH_3$ | CH | 1 | 148 |
| 286 | " | " | H | $OCH_3$ | $CH_3$ | N | 1 | 124-126 |
| 287 | " | " | H | $OC_2H_5$ | $NHCH_3$ | N | 1 | 167-169 |
| 288 | " | " | H | $OCH_3$ | $OCH_2CF_3$ | N | 1 | 115-117 |
| 289 | " | 6-$OC_2H_5$ | H | $OCH_3$ | $CH_3$ | CH | 1 | 157-158 |

| Bsp. Nr. | $R^1$ | $R^2$ | $R^3$ | $R^5$ | $R^6$ | E | n | Fp. |
|---|---|---|---|---|---|---|---|---|
| 290 | $CH_2CH_3$ | 6-$OC_2H_5$ | H | $OCH_3$ | $CH_3$ | N | 1 | 166-167 |
| 291 | " | " | H | $CH_3$ | $CH_3$ | CH | 1 | 139-140 |
| 292 | " | " | H | $OC_{H3}$ | $OCH_2CF_3$ | N | 1 | 124 |
| 293 | " | " | $CH_3$ | $OC_{H3}$ | $OCH_3$ | CH | 1 | 112-115 |
| 294 | " | " | $CH_3$ | $OCH_3$ | $CH_3$ | N | 1 | |
| 295 | " | " | H | $OCF_2H$ | $OCF_2H$ | CH | 1 | |
| 296 | " | " | H | $OCH_3$ | $OCH_3$ | N | 1 | 150-151 (Zers.) |
| 297 | " | 6-$CF_3$ | H | $OCH_3$ | $OCH_3$ | CH | 1 | 181 |
| 298 | " | " | H | $OCH_3$ | $CH_3$ | CH | 1 | 151-153 |
| 299 | " | " | H | $CH_3$ | $CH_3$ | CH | 1 | |
| 300 | " | " | H | $OCH_3$ | $OCH_2CF_3$ | N | 1 | |
| 301 | " | " | $CH_3$ | $OCH_3$ | $OCH_3$ | CH | 1 | |
| 302 | " | " | $CH_3$ | $OCH_3$ | $CH_3$ | N | 1 | |
| 303 | " | " | H | $OCF_2H$ | $OCF_2H$ | CH | 1 | |
| 304 | " | " | H | $OCH_3$ | $OCH_3$ | N | 1 | |
| 305 | " | 6-$CH_3$ | $CH_3$ | $OCH_3$ | $OCH_3$ | CH | 1 | 88 (Zers.) |
| 306 | " | 5-$CH_3$ | H | $OCH_3$ | $OCH_3$ | CH | 1 | 158-161 |
| 307 | " | " | H | $OCH_3$ | $CH_3$ | CH | 1 | 113 (Zers.) |
| 308 | " | 5-$C_2H_5$ | H | $OCH_3$ | $OCH_3$ | CH | 1 | |
| 309 | " | " | H | $OCH_3$ | $CH_3$ | CH | 1 | |
| 310 | " | 5-Cl | H | $OCH_3$ | $OCH_3$ | CH | 1 | |
| 311 | " | " | H | $OCH_3$ | $CH_3$ | CH | 1 | |
| 312 | " | 5-$OCH_3$ | H | $OCH_3$ | $OCH_3$ | CH | 1 | |
| 313 | " | " | H | $OCH_3$ | $CH_3$ | CH | 1 | |
| 314 | " | 6-$CO_2CH_3$ | $CH_3$ | $OCH_3$ | $OCH_3$ | CH | 1 | 102-105 (Zers.) |
| 315 | " | " | H | $OCH_3$ | $OCH_3$ | N | 1 | 154-155 |
| 316 | $CH_2CH_2CH_3$ | 6-$C_2H_5$ | H | $OCH_3$ | $OCH_3$ | CH | 1 | |
| 317 | " | " | H | $OCH_3$ | $CH_3$ | CH | 1 | |
| 318 | " | " | H | $CH_3$ | $CH_3$ | CH | 1 | |
| 319 | " | " | $CH_3$ | $OCH_3$ | $OCH_3$ | CH | 1 | |
| 320 | " | " | $CH_3$ | $OCH_3$ | $CH_3$ | N | 1 | |
| 321 | " | " | H | $OCF_2H$ | $OCF_2H$ | CH | 1 | |

| Bsp. Nr. | $R^1$ | $R^2$ | $R^3$ | $R^5$ | $R^6$ | E | n | Fp. |
|---|---|---|---|---|---|---|---|---|
| 322 | " | 6-$C_3H_7$ | H | $OCH_3$ | $OCH_3$ | CH | 1 | 143-145 |
| 323 | " | " | H | $OCH_3$ | $CH_3$ | CH | 1 | |
| 324 | " | " | H | $OCH_3$ | $CH_3$ | N | 1 | |
| 325 | " | 6-$OC_2H_5$ | H | $OCH_3$ | $OCH_3$ | CH | 1 | 138-139 |
| 326 | " | " | H | $OCH_3$ | $CH_3$ | N | 1 | |
| 327 | " | " | H | $CH_3$ | $CH_3$ | CH | 1 | |
| 328 | " | " | $CH_3$ | $OCH_3$ | $OCH_3$ | CH | 1 | 111-113 |
| 329 | " | " | $CH_3$ | $OCH_3$ | $CH_3$ | N | 1 | |
| 330 | " | " | H | $OCF_2H$ | $OCF_2H$ | CH | 1 | |
| 331 | " | 6-$CF_3$ | H | $OCH_3$ | $OCH_3$ | CH | 1 | 165-166 |
| 332 | " | " | H | $OCH_3$ | $CH_3$ | CH | 1 | |
| 333 | " | " | H | $CH_3$ | $CH_3$ | CH | 1 | |
| 334 | " | " | $CH_3$ | $OCH_3$ | $OCH_3$ | CH | 1 | |
| 335 | " | " | H | $OCF_2H$ | $OCF_2H$ | CH | 1 | 121-122 |
| 336 | " | 6-$CH_3$ | $CH_3$ | $OCH_3$ | $OCH_3$ | CH | 1 | |
| 337 | " | 6-$CO_2CH_3$ | $CH_3$ | $OCH_3$ | $OCH_3$ | CH | 1 | |
| 338 | $CH(CH_3)_2$ | 6-$C_2H_5$ | H | $OCH_3$ | $OCH_3$ | CH | 1 | |
| 339 | " | " | H | $OCH_3$ | $CH_3$ | CH | 1 | |
| 340 | " | " | H | $CH_3$ | $CH_3$ | CH | 1 | |
| 341 | " | " | $CH_3$ | $OCH_3$ | $OCH_3$ | CH | 1 | |
| 342 | " | " | $CH_3$ | $OCH_3$ | $CH_3$ | N | 1 | |
| 343 | " | " | H | $OCF_2H$ | $OCF_2H$ | CH | 1 | |
| 344 | " | 6-$C_3H_7$ | H | $OCH_3$ | $OCH_3$ | CH | 1 | 137 |
| 345 | " | " | H | $OCH_3$ | $CH_3$ | CH | 1 | 131-133 |
| 346 | " | " | H | $OCH_3$ | $CH_3$ | N | 1 | |
| 347 | " | 6-$OC_2H_5$ | H | $OCH_3$ | $OCH_3$ | CH | 1 | 129-130 |
| 348 | " | " | H | $OCH_3$ | $CH_3$ | CH | 1 | |
| 349 | " | " | H | $OCH_3$ | $CH_3$ | N | 1 | |
| 350 | " | " | H | $CH_3$ | $CH_3$ | CH | 1 | |
| 351 | " | " | $CH_3$ | $OCH_3$ | $OCH_3$ | CH | 1 | |

| Bsp. Nr. | $R^1$ | $R^2$ | $R^3$ | $R^5$ | $R^6$ | E | n | Fp. |
|---|---|---|---|---|---|---|---|---|
| 352 | " | " | $CH_3$ | $OCH_3$ | $CH_3$ | N | 1 | |
| 353 | " | " | H | $OCF_2H$ | $OCF_2H$ | CH | 1 | |
| 354 | " | 6-$CF_3$ | H | $OCH_3$ | $OCH_3$ | CH | 1 | 157-159 |
| 355 | " | " | H | $OCH_3$ | $CH_3$ | CH | 1 | |
| 356 | " | " | H | $CH_3$ | $CH_3$ | CH | 1 | |
| 357 | " | " | $CH_3$ | $OCH_3$ | $OCH_3$ | CH | 1 | |
| 358 | " | " | H | $OCF_2H$ | $OCF_2H$ | CH | 1 | |
| 359 | " | 6-$CH_3$ | $CH_3$ | $OCH_3$ | $OCH_3$ | CH | 1 | |
| 360 | " | 6-$CO_2CH_3$ | $CH_3$ | $OCH_3$ | $OCH_3$ | CH | 1 | |
| 361 | $CH_2CH_3$ | 3,4,6-$F_3$ | H | $OCH_3$ | $OCH_3$ | CH | 3 | |
| 362 | " | 6-Cl | $C_2H_5$ | $OCH_3$ | $OCH_3$ | CH | 1 | |
| 363 | " | 6-$OCH_3$ | $C_2H_5$ | $OCH_3$ | $OCH_3$ | CH | 1 | |
| 364 | " | " | $C_2H_5$ | $OCH_3$ | $OCH_3$ | N | 1 | |
| 365 | $CH_2CH_2CH_2$ | " | $C_2H_5$ | $OCH_3$ | $OCH_3$ | N | 1 | |

**Tabelle 2**

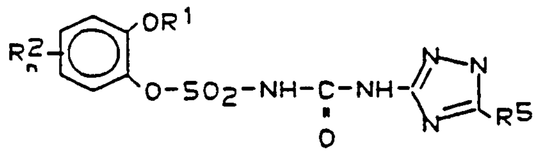

| Bsp.Nr. | $R^1$ | $R^2$ | $R^5$ | n | Fp.[°C] |
|---|---|---|---|---|---|
| 366 | $CH_2CH_3$ | H | $CH_3$ | 0 | |
| 367 | $CH_2CH_3$ | H | H | 0 | |
| 368 | $CH_2CH_3$ | H | $OCH_3$ | 0 | |
| 369 | $CH_2CH_2CH_3$ | H | $CH_3$ | 0 | |
| 370 | $CH_2CH_2CH_3$ | H | H | 0 | |
| 371 | $CH_2CH_2CH_3$ | H | $OCH_3$ | 0 | |
| 372 | $CH(CH_3)_2$ | H | $CH_3$ | 0 | |
| 373 | $CH(CH_3)_2$ | H | H | 0 | |
| 374 | $CH(CH_3)_2$ | H | $OCH_3$ | 0 | |
| 375 | $CH_2CH_3$ | 6-$CH_3$ | $OCH_3$ | 1 | |
| 376 | $CH_2CH_2CH_3$ | 6-$OCH_3$ | $OCH_3$ | 1 | |
| 377 | $CH(CH_3)_2$ | 6-Cl | $OCH_3$ | 1 | |
| 378 | $CH_2CH_3$ | 6-$CF_3$ | $OCH_3$ | 1 | |
| 379 | $CH_2CH_2CH_3$ | 6-F | $OCH_3$ | 1 | |
| 380 | $CH(CH_3)_2$ | 6-$OCF_2H$ | $OCH_3$ | 1 | |

**Tabelle 3**

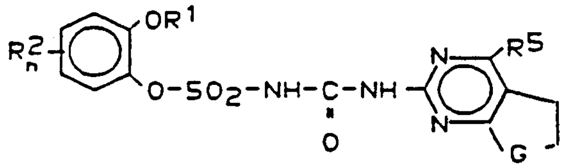

| Bsp.Nr. | $R^1$ | $R^2$ | $R^5$ | G | n | Fp.[°C] |
|---|---|---|---|---|---|---|
| 381 | $CH_2CH_3$ | H | $CH_3$ | $CH_2$ | 0 | |
| 382 | $CH_2CH_3$ | H | H | $CH_2$ | 0 | |
| 383 | $CH_2CH_3$ | H | $OCH_3$ | $CH_2$ | 0 | |
| 384 | $CH_2CH_2CH_3$ | H | $CH_3$ | $CH_2$ | 0 | |
| 385 | $CH_2CH_2CH_3$ | H | H | $CH_2$ | 0 | |
| 386 | $CH_2CH_2CH_3$ | H | $OCH_3$ | $CH_2$ | 0 | |
| 387 | $CH(CH_3)_2$ | H | $CH_3$ | $CH_2$ | 0 | |
| 388 | $CH(CH_3)_2$ | H | H | $CH_2$ | 0 | |
| 389 | $CH(CH_3)_2$ | H | $OCH_3$ | $CH_2$ | 0 | |
| 390 | $CH_2CH_3$ | H | $CH_3$ | O | 0 | |
| 391 | $CH_2CH_3$ | H | H | O | 0 | |
| 392 | $CH_2CH_3$ | H | $OCH_3$ | O | 0 | |
| 393 | $CH_2CH_2CH_3$ | H | $CH_3$ | O | 0 | |
| 394 | $CH_2CH_2CH_3$ | H | H | O | 0 | |
| 395 | $CH_2CH_2CH_3$ | H | $OCH_3$ | O | 0 | |
| 396 | $CH(CH_3)_2$ | H | $CH_3$ | O | 0 | |
| 397 | $CH(CH_3)_2$ | H | H | O | 0 | |
| 398 | $CH(CH_3)_2$ | H | $OCH_3$ | O | 0 | |
| 399 | $CH_2CH_3$ | 6-$CH_3$ | $CH_3$ | $CH_2$ | 1 | |
| 400 | $CH_2CH_2CH_3$ | 6-$OCH_3$ | $CH_3$ | O | 1 | |
| 401 | $CH(CH_3)_2$ | 6-Cl | $CH_3$ | $CH_2$ | 1 | |
| 402 | $CH_2CH_3$ | 6-$CF_3$ | $CH_3$ | O | 1 | |
| 403 | $CH_2CH_2CH_3$ | 6-F | $CH_3$ | $CH_2$ | 1 | |
| 404 | $CH(CH_3)_2$ | 6-$OCF_2H$ | $CH_3$ | O | 1 | |

**Tabelle 4**

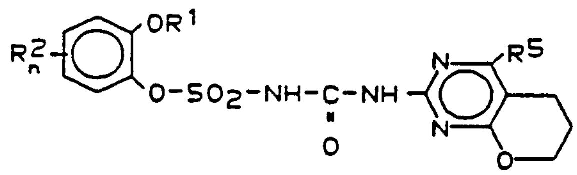

| Bsp.Nr. | $R^1$ | $R^2$ | $R^5$ | n | Fp. |
|---|---|---|---|---|---|
| 405 | $CH_2CH_3$ | H | $CH_3$ | 0 | |
| 406 | $CH_2CH_3$ | H | H | 0 | |
| 407 | $CH_2CH_3$ | H | $OCH_3$ | 0 | |
| 408 | $CH_2CH_2CH_3$ | H | $CH_3$ | 0 | |
| 409 | $CH_2CH_2CH_3$ | H | H | 0 | |
| 410 | $CH_2CH_2CH_3$ | H | $OCH_3$ | 0 | |
| 411 | $CH(CH_3)_2$ | H | $CH_3$ | 0 | |
| 412 | $CH(CH_3)_2$ | H | H | 0 | |
| 413 | $CH(CH_3)_2$ | H | $OCH_3$ | 0 | |
| 414 | $CH_2CH_3$ | 6-$CH_3$ | $OCH_3$ | 1 | |
| 415 | $CH_2CH_2CH_3$ | 6-$OCH_3$ | $OCH_3$ | 1 | |
| 416 | $CH(CH_3)_2$ | 6-Cl | $OCH_3$ | 1 | |
| 417 | $CH_2CH_3$ | 6-$CF_3$ | $OCH_3$ | 1 | |
| 418 | $CH_2CH_2CH_3$ | 6-F | $OCH_3$ | 1 | |
| 419 | $CH(CH_3)_2$ | 6-$OCF_2H$ | $OCH_3$ | 1 | |

Tabelle 5

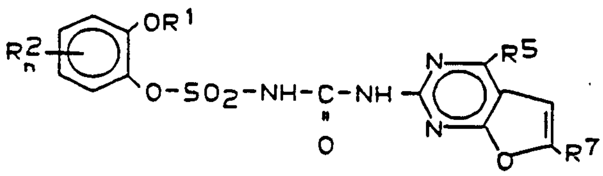

| Bsp.Nr. | $R^1$ | $R^2$ | $R^5$ | R7 | n | Fp. |
|---|---|---|---|---|---|---|
| 420 | $CH_2CH_3$ | H | $CH_3$ | $CH_3$ | 0 | |
| 421 | $CH_2CH_3$ | H | H | $CH_3$ | 0 | |
| 422 | $CH_2CH_3$ | H | $OCH_3$ | $CH_3$ | 0 | |
| 423 | $CH_2CH_3$ | H | $CH_3$ | H | 0 | |
| 424 | $CH_2CH_2CH_3$ | H | $CH_3$ | $CH_3$ | 0 | |
| 425 | $CH_2CH_2CH_3$ | H | H | $CH_3$ | 0 | |
| 426 | $CH_2CH_2CH_3$ | H | $OCH_3$ | $CH_3$ | 0 | |
| 427 | $CH_2CH_2CH_3$ | H | $CH_3$ | H | 0 | |
| 428 | $CH(CH_3)_2$ | H | $CH_3$ | $CH_3$ | 0 | |
| 429 | $CH(CH_3)_2$ | H | H | $CH_3$ | 0 | |
| 430 | $CH(CH_3)_2$ | H | $OCH_3$ | $CH_3$ | 0 | |
| 431 | $CH(CH_3)_2$ | H | $CH_3$ | H | 0 | |
| 432 | $CH_2CH_3$ | 6-$CH_3$ | $CH_3$ | H | 1 | |
| 433 | $CH_2CH_2CH_3$ | 6-$OCH_3$ | $CH_3$ | $CH_3$ | 1 | |
| 434 | $CH(CH_3)_2$ | 6-Cl | $CH_3$ | H | 1 | |
| 435 | $CH_2CH_3$ | 6-$CF_3$ | $CH_3$ | $CH_3$ | 1 | |
| 436 | $CH_2CH_2CH_3$ | 6-F | $CH_3$ | H | 1 | |
| 437 | $CH(CH_3)_2$ | 6-$OCF_2H$ | $CH_3$ | $CH_3$ | 1 | |

Tabelle 6

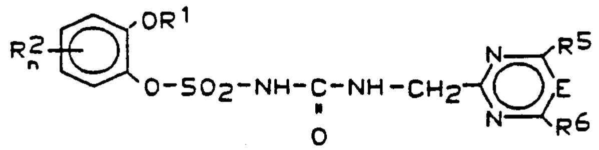

| Bsp.Nr. | $R^1$ | $R^2$ | $R^5$ | $R^6$ | E | n | Fp. |
|---|---|---|---|---|---|---|---|
| 438 | $CH_2CH_3$ | H | $OCH_3$ | $OCH_3$ | CH | 0 | |
| 439 | $CH_2CH_3$ | H | $OCH_3$ | $CH_3$ | CH | 0 | |
| 440 | $CH_2CH_3$ | H | $OCH_3$ | $CH_3$ | N | 0 | |
| 441 | $CH_2CH_2CH_3$ | H | $OCH_3$ | $OCH_3$ | CH | 0 | |
| 442 | $CH_2CH_2CH_3$ | H | $OCH_3$ | $CH_3$ | CH | 0 | |
| 443 | $CH_2CH_2CH_3$ | H | $OCH_3$ | $CH_3$ | N | 0 | |
| 444 | $CH(CH_3)_2$ | H | $OCH_3$ | $OCH_3$ | CH | 0 | |
| 445 | $CH(CH_3)_2$ | H | $OCH_3$ | $CH_3$ | CH | 0 | |
| 446 | $CH(CH_3)_2$ | H | $OCH_3$ | $CH_3$ | N | 1 | |
| 447 | $CH_2CH_3$ | 6-$CH_3$ | $OCH_3$ | $CH_3$ | N | 1 | |
| 448 | $CH_2CH_2CH3$ | 6-$OCH_3$ | $OCH_3$ | $OCH_3$ | CH | 1 | |
| 449 | $CH(CH_3)_2$ | 6-Cl | $OCH_3$ | $OCH_3$ | N | 1 | |
| 450 | $CH_2CH_3$ | 6-$CF_3$ | $OCH_3$ | $CH_3$ | CH | 1 | |
| 451 | $CH_2CH_2CH_3$ | 6-F | $OCH_3$ | $OCH_3$ | N | 1 | |
| 452 | $CH(CH_3)_2$ | 6-$OCF_2H$ | $OCH_3$ | $CH_3$ | CH | 1 | |

Tabelle 7

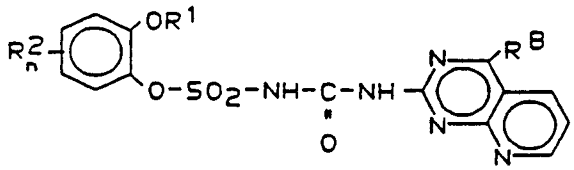

| Bsp.Nr. | $R^1$ | $R^2$ | $R^8$ | n | Fp. |
|---|---|---|---|---|---|
| 453 | $CH_2CH_3$ | H | $OCH_3$ | 0 | |
| 454 | $CH_2CH_3$ | H | $CH_3$ | 0 | |
| 455 | $CH_2CH_2CH_3$ | H | $OCH_3$ | 0 | |
| 456 | $CH_2CH_2CH_3$ | H | $CH_3$ | 0 | |
| 457 | $CH(CH_3)_2$ | H | $OCH_3$ | 0 | |
| 458 | $CH(CH_3)_2$ | H | $CH_3$ | 0 | |
| 459 | $CH_2CH_3$ | 6-$CH_3$ | $CH_3$ | 1 | |
| 460 | $CH_2CH_2CH_3$ | 6-$OCH_3$ | $CH_3$ | 1 | |
| 461 | $CH(CH_3)_2$ | 6-Cl | $OCH_3$ | 1 | |
| 462 | $CH_2CH_3$ | 6-$CF_3$ | $CH_3$ | 1 | |
| 463 | $CH_2CH_2CH_3$ | 6-F | $OCH_3$ | 1 | |
| 464 | $CH(CH_3)_2$ | 6-$OCF_2H$ | $CH_3$ | 1 | |

Tabelle 8

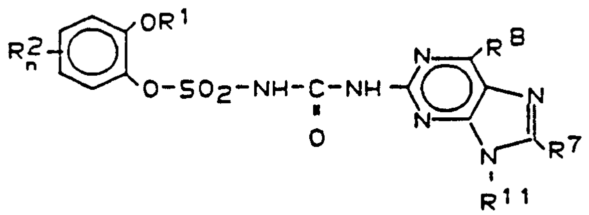

| Bsp.Nr. | $R^1$ | $R^2$ | $R^7$ | $R^8$ | $R^{11}$ | n | Fp. |
|---|---|---|---|---|---|---|---|
| 465 | $CH_2CH_3$ | H | H | $OCH_3$ | H | 0 | |
| 466 | $CH_2CH_3$ | H | H | $CH_3$ | $CH_3$ | 0 | |
| 467 | $CH_2CH_3$ | H | $CH_3$ | $CH_3$ | $CH_3$ | 0 | |
| 468 | $CH_2CH_2CH_3$ | H | H | $OCH_3$ | H | 0 | |
| 469 | $CH_2CH_2CH_3$ | H | H | $CH_3$ | $CH_3$ | 0 | |
| 470 | $CH_2CH_2CH_3$ | H | $CH_3$ | $CH_3$ | $CH_3$ | 0 | |
| 471 | $CH(CH_3)_2$ | H | H | $OCH_3$ | H | 0 | |
| 472 | $CH(CH_3)_2$ | H | H | $CH_3$ | $CH_3$ | 0 | |
| 473 | $CH(CH_3)_2$ | H | $CH_3$ | $CH_3$ | $CH_3$ | 0 | |
| 474 | $CH_2CH_3$ | 6-$CH_3$ | H | $CH_3$ | H | 1 | |
| 475 | $CH_2CH_2CH_3$ | 6-$OCH_3$ | H | $CH_3$ | $CH_3$ | 1 | |
| 476 | $CH(CH_3)_2$ | 6-Cl | $CH_3$ | $OCH_3$ | $CH_3$ | 1 | |
| 477 | $CH_2CH_3$ | 6-$CF_3$ | H | $CH_3$ | H | 1 | |
| 478 | $CH_2CH_3$ | 6-F | H | $OCH_3$ | $CH_3$ | 1 | |
| 479 | $CH(CH_3)_2$ | 6-Cl | $CH_3$ | $CH_3$ | $CH_3$ | 1 | |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

**1. Unkrautwirkung im Vorauflauf**

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu

unbehandelten Kontrollen. Wie die Boniturwerte in Tabelle 9 zeigen, weisen die erfindungsgemäßen Verbindungen eine gute herbizide Vorauflaufwirksamkeit gegen ein breites Spektrum von Ungräsern und Unkräutern auf.

**2. Unkrautwirkung im Nachauflauf**

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 10).

**3. Kulturpflanzenverträglichkeit**

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

Tabelle 9

| Vorauflaufwirkung der erfindungsgemäßen Verbindungen | | | | | | | |
|---|---|---|---|---|---|---|---|
| Bsp. Nr. Nr. | Dosierung kg a.i./ha | herbizide Wirkung | | | | | |
| | | SIA | CRS | STM | AS | ECG | LOM |
| 2 | 0.6 | 5 | 5 | 5 | 1 | 3 | 4 |
| 3 | 0,6 | 5 | 5 | 5 | 2 | 3 | 3 |
| 4 | 0,6 | 5 | 5 | 4 | 4 | 4 | 1 |
| 5 | 0,6 | 5 | 4 | 4 | 2 | 2 | 1 |
| 7 | 0,6 | 5 | 5 | 3 | 2 | 2 | 2 |
| 67 | 0,6 | 5 | 5 | 5 | 2 | 2 | 2 |
| 92 | 0,3 | 5 | 5 | 5 | 1 | 3 | 1 |
| 114 | 0,3 | 5 | 5 | 5 | 4 | 5 | 5 |
| 117 | 0,3 | 5 | 5 | 5 | 2 | 4 | 2 |
| 118 | 0,3 | 5 | 5 | 5 | 5 | 4 | 5 |
| 129 | 0,3 | 5 | 5 | 5 | 3 | 3 | 4 |
| 137 | 0,3 | 5 | 5 | 5 | 4 | 4 | 5 |
| 257 | 0,3 | 5 | 5 | 5 | 1 | 2 | 1 |

Tabelle 10

| Nachlaufwirkung der erfindungsgemäßen Verbindungen | | | | | | | | |
|---|---|---|---|---|---|---|---|---|
| Bsp. Nr. Nr. | Dosierung kg a.i./ha | herbizide Wirkung | | | | | | |
| | | SIA | CRS | STM | AS | ECG | LOM |
| 2 | 0,6 | 5 | 5 | 5 | 0 | 4 | 5 |
| 3 | 0,6 | 5 | 5 | 5 | 1 | 4 | 4 |
| 4 | 0,6 | 5 | 4 | 4 | 3 | 4 | 2 |
| 5 | 0,6 | 5 | 5 | 4 | 2 | 2 | 3 |
| 7 | 0,6 | 5 | 5 | 5 | 1 | 3 | 2 |
| 67 | 0,6 | 5 | 5 | 5 | 0 | 5 | 3 |
| 92 | 0,3 | 5 | 5 | 5 | 3 | 4 | 3 |
| 114 | 0,3 | 5 | 5 | 5 | 3 | 5 | 5 |
| 117 | 0,3 | 5 | 4 | 5 | 2 | 4 | 2 |
| 118 | 0,3 | 5 | 5 | 5 | 4 | 5 | 4 |
| 129 | 0,3 | 5 | 4 | 5 | 2 | 4 | 2 |
| 137 | 0,3 | 5 | 5 | 5 | 4 | 4 | 5 |
| 257 | 0,3 | 4 | 5 | 5 | 1 | 3 | 1 |

Abkürzungen:
SIA = Sinapis alba
CRS = Chrysanthemum segetum
STM = Stellaria media
AS = Avena sativa
ECG = Echinochloa crus-galli
LOM = Lolium multiflorum
a.i. = Aktivsubstanz

**Patentansprüche**
**Patentansprüche für folgende Vertragsstaaten : BE, CH, DE, FR, GB, IT, LI, NL, SE**

**1.** Verbindungen der Formel I oder deren Salze

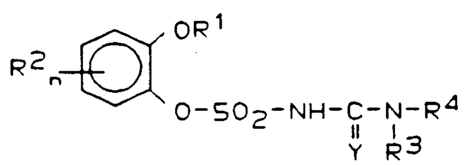

(I),

worin

| | |
|---|---|
| $R^1$ | Ethyl, Propyl oder Isopropyl, |
| $R^2$ | Halogen, $NO_2$, $CF_3$, CN, $(C_1-C_4)$Alkyl, $(C_1-C_4)$Alkoxy, $(C_1-C_4)$Alkylthio oder $(C_1-C_4$-Alkoxy)carbonyl, |
| n | 0, 1, 2 oder 3, |
| Y | O oder S, |
| $R^3$ | Wasserstoff, $(C_1-C_8)$Alkyl, $(C_2-C_8)$Alkenyl, $(C_2-C_8)$Alkinyl oder $(C_1-C_4)$Alkoxy; |
| $R^4$ | einen heterocyclischen Rest der Formeln |

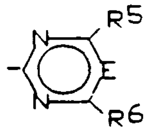 , 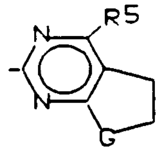 , 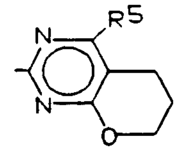 ,

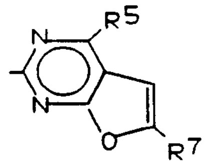 , 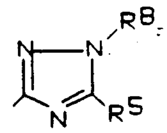 , 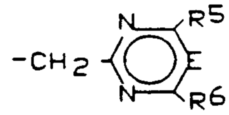 ,

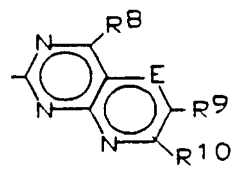 oder 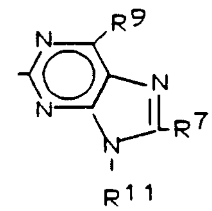

E CH oder N,
G O oder $CH_2$,
$R^5$, $R^6$ unabhängig voneinander Wasserstoff, $(C_1-C_6)$-Alkyl, $(C_1-C_6)$Alkoxy oder $(C_1-C_6)$Alkylthio, wobei die vorgenannten alkylhaltigen Reste ein- oder mehrfach durch Halogen oder ein- oder zweifach durch $(C_1-C_4)$Alkoxy oder $(C_1-C_4)$Alkylthio substituiert sein können, ferner einen Rest der Formel $-NR^{12}R^{13}$, $-OCHR^7-CO_2R^{12}$, $(C_3-C_6)$Cycloalkyl, $(C_3-C_5)$Alkenyl, $(C_2-C_4)$Alkinyl, $(C_3-C_5)$- Alkenyloxy oder $(C_3-C_5)$Alkinyloxy,
$R^7$ Wasserstoff oder $(C_1-C_4)$Alkyl,
$R^8$ $(C_1-C_4)$Alkyl, $-CHF_2$ oder $-CH_2CF_3$,
$R^9$,$R^{10}$ unabhängig voneinander Wasserstoff, $(C_1-C_4)$Alkyl, $(C_1-C_5)$Alkoxy oder Halogen,
$R^{11}$ Wasserstoff, $(C_1-C_4)$Alkyl, $-CHF_2$ oder $CH_2CF_3$ und
$R^{12}$,$R^{13}$ unabhängig voneinander Wasserstoff, $(C_1-C_4)$Alkyl, $(C_2-C_4)$Alkenyl oder $(C_3-C_4)$Alkinyl

bedeuten.

**2.** Verbindungen der Formel I von Anspruch 1 oder deren Salze, worin
n = 0 oder 1
Y = O
$R^1$ = Ethyl, Propyl oder Isopropyl,
$R^2$ = in Position 6 des Phenylringes orientiert ist und Fluor, Chlor, $(C_1-C_4)$Alkyl, $(C_1-C_4)$-Alkoxy oder $(C_1-C_4)$Alkoxycarbonyl bedeutet,
$R^3$ = H, $(C_1-C_4)$Alkyl oder $(C_3-C_4)$Alkenyl,
$R^4$ = einen Rest der Formel,

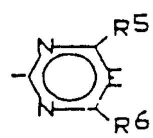

E = CH oder N und
$R^5$,$R^6$ = $(C_1-C_4)$Alkyl, $(C_1-C_4)$Alkoxy oder $(C_1-C_4)$Alkylthio, wobei die vorgenannten Reste im Alkylteil ein- oder mehrfach durch Halogen oder ein- oder zweifach durch $(C_1-C_4)$-Alkoxy oder $(C_1-C_4)$Alkylthio substituiert sein können, bedeuten.

**3.** Verbindungen der Formel I von Anspruch 2 oder deren Salze, dadurch gekennzeichnet, daß $R^1$ = Ethyl, n = O, Y = Sauerstoff, $R^3$ = Wasserstoff,

E = CH und
$R^5$, $R^6$ unabhängig voneinander Methyl oder Methoxy bedeuten.

**4.** Verfahren zur Herstellung der Verbindungen der Formel I oder deren Salze gemäß einem der Ansprüche 1 bis 3, dadurch gekennzeichnet, daß man
(a) eine Verbindung der Formel (II)

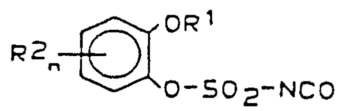

(II)

mit einer Verbindung der Formel (III)

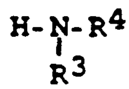

(III)

umsetzt, oder
(b) eine Verbindung der Formel (IV)

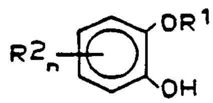

(IV)

mit einem Chlorsulfonylharnstoff der Formel (V)

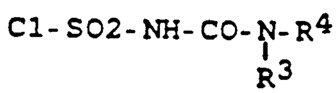

(V)

umsetzt, oder
(c) eine Verbindung der Formel (VI)

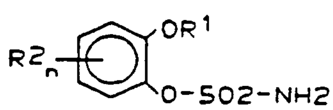

(VI)

mit einem Carbamat der Formel (VII)

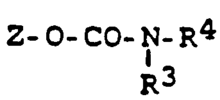

(VII)

worin
Z Phenyl oder $(C_1-C_6)$Alkyl bedeutet,
umsetzt und die erhaltenen Verbindungen gegebenenfalls in ihre Salze überführt.

**5.** Herbizide oder pflanzenwachstumsregulierende Mittel, dadurch gekennzeichnet, daß sie eine wirksame Menge einer oder mehrerer Verbindungen der Formel I von Anspruch 1 , 2 oder 3 oder deren Salze und inerte Hilfsstoffe enthalten.

**6.** Verwendung der Verbindungen der Formel I von Anspruch 1 , 2 oder 3 oder deren Salze als Herbizide oder Pflanzenwachstumsregulatoren.

**7.** Verfahren zur Bekämpfung von Schadpflanzen, dadurch gekennzeichnet, daß man auf diese oder die landwirtschaftlich oder industriell genutzten Böden eine wirksame Menge einer oder mehrerer Verbindungen der Formel I oder deren Salze von Anspruch 1, 2 oder 3 appliziert.

**8.** Verfahren zur Wachstumsregulierung von Nutzpflanzen, dadurch gekennzeichnet, daß man auf diese oder die Anbaufläche eine wirksame Menge einer oder mehrerer Verbindungen der Formel I von Anspruch 1, 2 oder 3 oder deren Salze appliziert.

**Patentansprüche für folgenden Vertragsstaat : ES**

**1.** Verfahren zur Bekämpfung von Schadpflanzen, dadurch gekennzeichnet, daß man auf diese oder die landwirtschaftlich oder industriell genutzten Böden eine wirksame Menge einer oder mehrerer Verbindungen der Formel I oder deren Salze

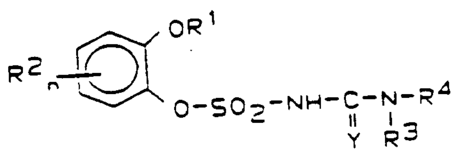

worin

| | |
|---|---|
| $R^1$ | Ethyl, Propyl oder Isopropyl, |
| $R^2$ | Halogen, $NO_2$, $CF_3$, CN, $(C_1-C_4)$Alkyl, $(C_1-C_4)$Alkoxy, $(C_1-C_4)$Alkylthio oder $(C_1-C_4$-Alkoxy)carbonyl, |
| n | 0, 1, 2 oder 3, |
| Y | O oder S, |
| $R^3$ | Wasserstoff, $(C_1-C_8)$Alkyl, $(C_2-C_8)$Alkenyl, $(C_2-C_8)$Alkinyl oder $(C_1-C_4)$Alkoxy; |
| $R^4$ | einen heterocyclischen Rest der Formeln |

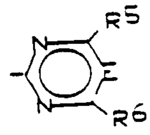, 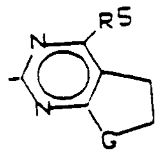, 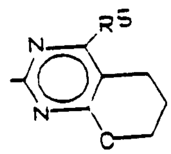,

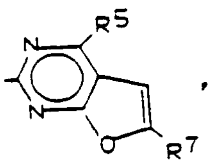, 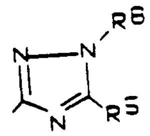, 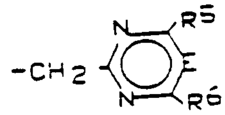,

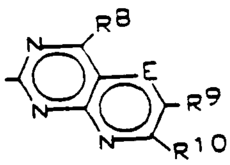 oder 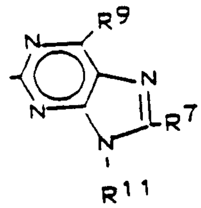

| | |
|---|---|
| E | CH oder N, |
| G | O oder $CH_2$, |
| $R^5$, $R^6$ | unabhängig voneinander Wasserstoff, $(C_1\text{-}C_6)$-Alkyl, $(C_1\text{-}C_6)$Alkoxy oder $(C_1\text{-}C_6)$Alkylthio, wobei die vorgenannten alkylhaltigen Reste ein- oder mehrfach durch Halogen oder ein- oder zweifach durch $(C_1\text{-}C_4)$Alkoxy oder $(C_1\text{-}C_4)$Alkylthio substituiert sein können, ferner einen Rest der Formel $-NR^{12}R^{13}$, $-OCHR^7\text{-}CO_2R^{12}$, $(C_3\text{-}C_6)$Cycloalkyl, $(C_3\text{-}C_5)$Alkenyl, $(C_2\text{-}C_4)$Alkinyl $(C_3\text{-}C_5)$-Alkenyloxy oder $(C_3\text{-}C_5)$Alkinyloxy, |
| $R^7$ | Wasserstoff oder $(C_1\text{-}C_4)$Alkyl, |
| $R^8$ | $(C_1\text{-}C_4)$Alkyl, $-CHF_2$ oder $-CH_2CF_3$, |
| $R^9$, $R^{10}$ | unabhängig voneinander Wasserstoff, $(C_1\text{-}C_4)$Alkyl, $(C_1\text{-}C_5)$Alkoxy oder Halogen, |
| $R^{11}$ | Wasserstoff, $(C_1\text{-}C_4)$Alkyl, $-CHF_2$ oder $CH_2CF_3$ und |
| $R^{12}$, $R^{13}$ | unabhängig voneinander Wasserstoff, $(C_1\text{-}C_4)$Alkyl, $(C_2\text{-}C_4)$Alkenyl oder $(C_3\text{-}C_4)$Alkinyl |

bedeuten, appliziert.

**2.** Verfahren nach Anspruch 1, dadurch gekennzeichnet, daß ein oder mehrere Verbindungen der Formel I, worin

| | |
|---|---|
| n = | 0 oder 1 |
| Y = | O |
| $R^1$ = | Ethyl, Propyl oder Isopropyl, |
| $R^2$ = | in Position 6 des Phenylringes orientiert ist und Fluor, Chlor, $(C_1\text{-}C_4)$Alkyl, $(C_1\text{-}C_4)$-Alkoxy oder $(C_1\text{-}C_4)$Alkoxycarbonyl bedeutet, |
| $R^3$ = | H, $(C_1\text{-}C_4)$Alkyl oder $(C_3\text{-}C_4)$Alkenyl, |
| $R^4$ = | einen Rest der Formel |

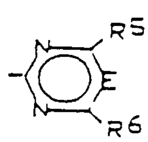

| | |
|---|---|
| E = | CH oder N und |
| $R^5$,$R^6$ = | $(C_1\text{-}C_4)$Alkyl, $(C_1\text{-}C_4)$Alkoxy oder $(C_1\text{-}C_4)$Alkylthio, wobei die vorgenannten Reste im Alkylteil ein- oder mehrfach durch Halogen oder ein- oder zweifach durch $(C_1\text{-}C_4)$-Alkoxy oder $(C_1\text{-}C_4)$Alkylthio substituiert sein können, bedeuten, oder deren Salze |

appliziert werden.

**3.** Verfahren nach Anspruch 2, dadurch gekennzeichnet, daß $R^1$ = Ethyl, n = O, Y = Sauerstoff, $R^3$ = Wasserstoff, R = CH und $R^5$ und $R^6$ unabhängig voneinander Methyl oder Methoxy bedeuten.

**4.** Verfahren zur Herstellung der Verbindungen der in Anspruch 1, 2 oder 3 definierten Formel I oder deren Salze, dadurch gekennzeichnet, daß man
(a) eine Verbindung der Formel (II)

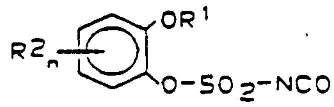

(II)

mit einer Verbindung der Formel (III)

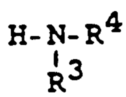

(III)

umsetzt, oder
(b) eine Verbindung der Formel (IV)

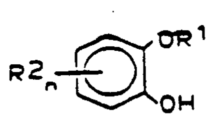

(IV)

mit einem Chlorsulfonylharnstoff der Formel (V)

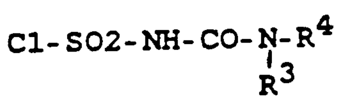

(V)

umsetzt, oder
(c) eine Verbindung der Formel (VI)

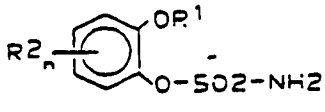

(VI)

mit einem Carbamat der Formel (VII)

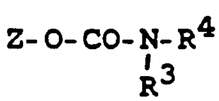

(VII)

worin

Z Phenyl oder ($C_1$-$C_6$)Alkyl bedeutet,
umsetzt und die erhaltenen Verbindungen gegebenenfalls in ihre Salze überführt.

5. Verfahren zur Wachstumsregulierung von Nutzpflanzen, dadurch gekennzeichnet, daß man auf diese oder die Anbaufläche eine wirksame Menge einer oder mehrerer Verbindungen der in Anspruch 1, 2 oder 3 definierten Formel I oder deren Salze appliziert.

## Claims

## Claims for the following Contracting States : BE, CH, DE, FR, GB, IT, LI, NL, SE

1. A compound of the formula I or salts thereof

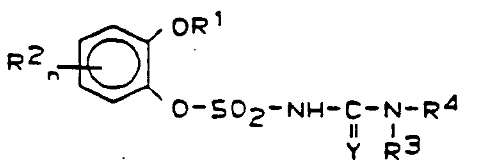

(I),

where

| | |
|---|---|
| $R^1$ | is ethyl, propyl or isopropyl, |
| $R^2$ | is halogen, $NO_2$, $CF_3$, CN, ($C_1$-$C_4$)alkyl, ($C_1$-$C_4$)alkoxy, ($C_1$-$C_4$)alkylthio or ($C_1$-$C_4$-alkoxy)carbonyl, |
| n | is 0, 1, 2 or 3, |
| Y | is O or S, |
| $R^3$ | is hydrogen, ($C_1$-$C_8$)alkyl, ($C_2$-$C_8$)alkenyl, ($C_2$-$C_8$)alkynyl or ($C_1$-$C_4$)alkoxy; |
| $R^4$ | is a heterocyclic radical of the formulae |

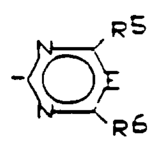 , 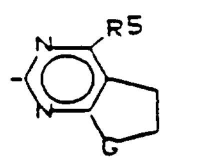 , 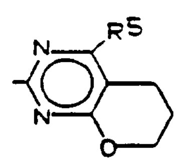 ,

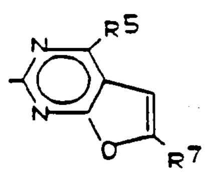 , 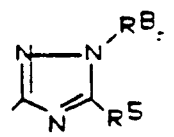 , 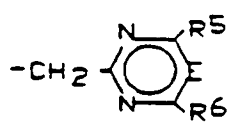 ,

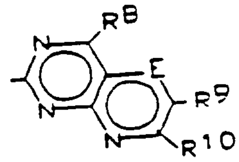

or

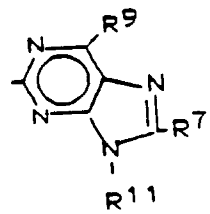

| | |
|---|---|
| E | is CH or N, |
| G | is O or $CH_2$, |
| $R^5$ and $R^6$ | independently of one another are hydrogen, $(C_1-C_6)$ alkyl, $(C_1-C_6)$alkoxy or $(C_1-C_6)$-alkylthio, it being possible for the abovementioned alkyl-containing radicals to be monosubstituted or polysubstituted by halogen or monosubstituted or disubstituted by $(C_1-C_4)$alkoxy or $(C_1-C_4)$alkylthio, furthermore are a radical of the formula $-NR^{12}R^{13}$, $-OCHR^7-CO_2R^{12}$, $(C_3-C_6)$cycloalkyl, $(C_3-C_5)$alkenyl, $(C_2-C_4)$alkynyl, $(C_3-C_5)$alkenyloxy or $(C_3-C_5)$alkynyloxy, |
| $R^7$ | is hydrogen or $(C_1-C_4)$alkyl, |
| $R^8$ | is $(C_1-C_4)$alkyl, $-CHF_2$ or $-CH_2CF_3$, |
| $R^9$ and $R^{10}$ | independently of one another are hydrogen, $(C_1-C_4)$alkyl, $(C_1-C_5)$alkoxy or halogen, |
| $R^{11}$ | is hydrogen, $(C_1-C_4)$alkyl, $-CHF_2$ or $CH_2CF_3$ and |
| $R^{12}$ and $R^{13}$ | independently of one another are hydrogen, $(C_1-C_4)$alkyl, $(C_2-C_4)$alkenyl or $(C_3-C_4)$-alkynyl. |

**2.** A compound of the formula I of claim 1 or salts thereof, where

| | |
|---|---|
| n = | 0 or 1 |
| Y | is O |
| $R^1$ | is ethyl, propyl or isopropyl, |
| $R^2$ | is orientated in the 6-position of the phenyl ring and is fluorine, chlorine, $(C_1-C_4)$alkyl, $(C_1-C_4)$alkoxy or $(C_1-C_4)$alkoxycarbonyl, |
| $R^3$ | is H, $(C_1-C_4)$alkyl or $(C_3-C_4)$alkenyl, |
| $R^4$ | is a radical of the formula |

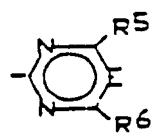

| | |
|---|---|
| E | is CH or N and |
| $R^5$ and $R^6$ | are $(C_1-C_4)$alkyl, $(C_1-C_4)$alkoxy or $(C_1-C_4)$alkylthio, it being possible for the abovementioned radicals to be monosubstituted or polysubstituted in the alkyl moiety by halogen or monosubstituted or disubstituted in the alkyl moiety by $(C_1-C_4)$alkoxy or $(C_1-C_4)$alkylthio. |

**3.** A compound of the formula I of claim 2 or salts thereof, wherein

| | |
|---|---|
| $R^4$ | is ethyl, n = 0, Y is oxygen, $R^3$ is hydrogen |
| E | is CH and |
| $R^5$ and $R^6$ | independently of one another are methyl or methoxy. |

**4.** A process for the preparation of a compound of the formula I or salts thereof, as claimed in one of claims 1 to 3, which comprises reacting

(a) a compound of the formula (II)

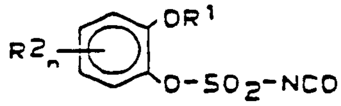 (II)

with a compound of the formula (III)

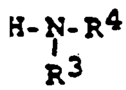 (III),

or
(b) a compound of the formula (IV)

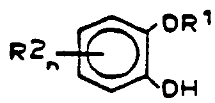 (IV)

with a chlorosulfonylurea of the formula (V)

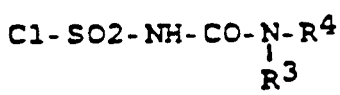 (V),

or
(c) a compound of the formula (VI)

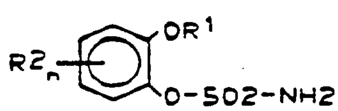 (VI)

with a carbamate of the formula (VII)

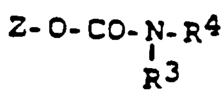 (VII)

where Z is phenyl or ($C_1$-$C_6$)alkyl and, if desired, converting the resulting compounds into their salts.

**5.** A herbicidal or plant growth-regulating composition, containing an effective amount of one or more compounds of the formula I of claim 1, 2 or 3 or of salts thereof, and inert auxiliaries.

**6.** The use of a compound of the formula I of claim 1, 2 or 3 or salts thereof as a herbicide or plant growth regulator.

**7.** A method of controlling noxious plants, which comprises applying an effective amount of one or more compounds of the formula I or salts thereof, of claim 1, 2 or 3, to these noxious plants or the soil used in agriculture or industry.

**8.** A method of regulating the growth of crop plants, which comprises applying an effective amount of one or more compounds of the formula I of claim 1, 2 or 3, or salts thereof, to these crop plants or the cropped area.

**Claims for the following Contracting State : ES**

**1.** A method of controlling noxious plants, which comprises applying to these noxious plants or the soil used in agriculture or industry an effective amount of one or more compounds of the formula I or salts thereof

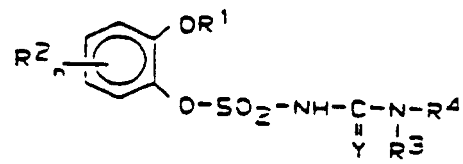

where

| | |
|---|---|
| $R^1$ | is ethyl, propyl or isopropyl, |
| $R^2$ | is halogen, $NO_2$, $CF_3$, CN, $(C_1-C_4)$alkyl, $(C_1-C_4)$alkoxy, $(C_1-C_4)$alkylthio or $(C_1-C_4$-alkoxy)carbonyl, |
| n | is 0, 1, 2 or 3, |
| Y | is O or S, |
| $R^3$ | is hydrogen, $(C_1-C_8)$alkyl, $(C_2-C_8)$alkenyl, $(C_2-C_8)$alkynyl or $(C_1-C_4)$alkoxy; |
| $R^4$ | is a heterocyclic radical of the formulae |

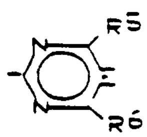 , 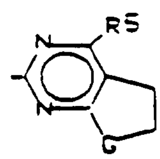 , 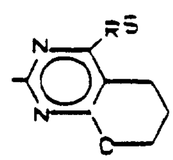 ,

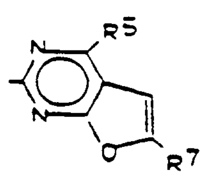 , 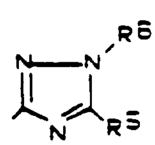 , 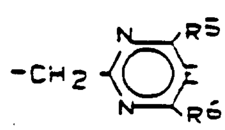 ,

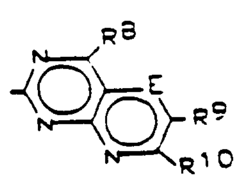

or

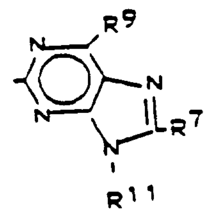

| | |
|---|---|
| E | is CH or N, |
| G | is O or $CH_2$, |
| $R^5$ and $R^6$ | independently of one another are hydrogen, $(C_1-C_6)$alkyl, $(C_1-C_6)$alkoxy or $(C_1-C_6)$-alkylthio, it being possible for the abovementioned alkyl-containing radicals to be monosubstituted or polysubstituted by halogen or monosubstituted or disubstituted by $(C_1-C_4)$alkoxy or $(C_1-C_4)$alkylthio, furthermore are a radical of the formula $-NR^{12}R^{13}$, $-OCHR^7-CO_2R^{12}$, $(C_3-C_6)$cycloalkyl, $(C_3-C_5)$alkenyl, $(C_2-C_4)$alkynyl, $(C_3-C_5)$alkenyloxy or $(C_3-C_5)$alkynyloxy, |
| $R^7$ | is hydrogen or $(C_1-C_4)$alkyl, |
| $R^8$ | is $(C_1-C_4)$alkyl, $-CHF_2$ or $-CH_2CF_3$, |
| $R^9$ and $R^{10}$ | independently of one another are hydrogen, $(C_1-C_4)$alkyl, $(C_1-C_5)$alkoxy or halogen, |
| $R^{11}$ | is hydrogen, $(C_1-C_4)$alkyl, $-CHF_2$ or $CH_2CF_3$ and |
| $R^{12}$ and $R^{13}$ | independently of one another are hydrogen, $(C_1-C_4)$alkyl, $(C_2-C_4)$alkenyl or $(C_3-C_4)$-alkynyl. |

**2.** The process as claimed in claim 1 which comprises applying one or more compounds of the formula I, where

| | |
|---|---|
| n = | 0 or 1 |
| Y | is O |
| $R^1$ | is ethyl, propyl or isopropyl, |
| $R^2$ | is orientated in the 6-position of the phenyl ring and is fluorine, chlorine, $(C_1-C_4)$alkyl, $(C_1-C_4)$alkoxy or $(C_1-C_4)$alkoxycarbonyl, |
| $R^3$ | is H, $(C_1-C_4)$alkyl or $(C_3-C_4)$alkenyl, |
| $R^4$ | is a radical of the formula |

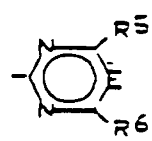

| | |
|---|---|
| E | is CH or N and |
| $R^5$ and $R^6$ | are $(C_1-C_4)$alkyl, $(C_1-C_4)$alkoxy or $(C_1-C_4)$-alkylthio, it being possible for the abovementioned radicals to be monosubstituted or polysubstituted in the alkyl moiety by halogen or monosubstituted or disubstituted in the alkyl moiety by $(C_1-C_4)$alkoxy or $(C_1-C_4)$alkylthio, or salts thereof. |

**3.** The process as claimed in claim 2, wherein $R^1$ is ethyl, n = 0, Y is oxygen, $R^3$ is hydrogen, E is CH and $R^5$ and $R^6$ independently of one another are methyl or methoxy.

**4.** A process for the preparation of a compound of the formula I as defined in claim 1, 2 or 3, or salts thereof, which comprises reacting

(a) a compound of the formula (II)

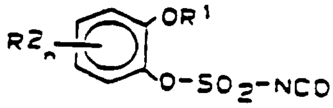

(II)

with a compound of the formula (III)

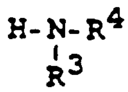

(III),

or

(b) a compound of the formula (IV)

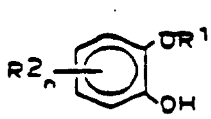

(IV)

with a chlorosulfonylurea of the formula (V)

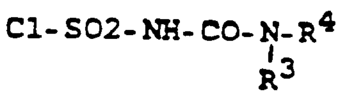

(V),

or

(c) a compound of the formula (VI)

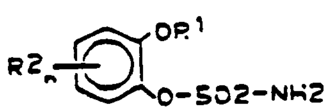

(VI)

with a carbamate of the formula (VII)

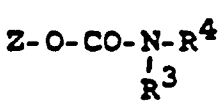

(VII)

where Z is phenyl or ($C_1$-$C_6$)alkyl and, if desired, converting the resulting compounds into their salts.

**5.** A method of regulating the growth of crop plants, which comprises applying an effective amount of one or more compounds of the formula I as defined in claim 1, 2 or 3, or salts thereof, to these crop plants or the cropped area.

## Revendications

## Revendications pour les Etats contractants suivants : BE, CH, DE, FR, GB, IT, LI, NL, SE

**1.** Composes de formule I ci-dessous et sels de ces composés,

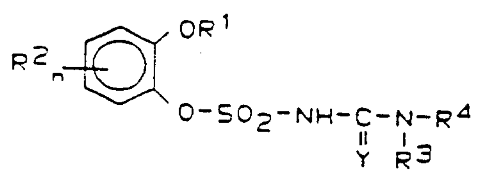

(I),

formule dans laquelle

| | |
|---|---|
| $R^1$ | représente le groupe éthyle, propyle ou isopropyle, |
| $R^2$ | un halogène ou un groupe $NO_2$, $CF_3$, CN, $(C_1\text{-}C_4)$alkyle, $(C_1\text{-}C_4)$alcoxy, $(C_1\text{-}C_4)$-alkylthio ou $(C_1\text{-}C_4$-alcoxy)carbonyle |
| n | le nombre 0, 1, 2 ou 3 |
| Y | l'oxygène ou le soufre, |
| $R^3$ | l'hydrogène ou bien un $(C_1\text{-}C_8)$alkyle, $(C_2\text{-}C_8)$alcényle, $(C_2\text{-}C_8)$alcynyle ou un $(C_1\text{-}C_4$-alcoxy), et |
| $R^4$ | un radical hétérocyclique de l'une des formules suivantes |

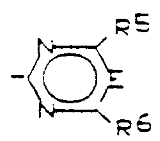
,
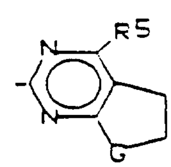
,
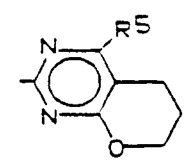
,

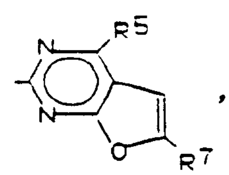
,
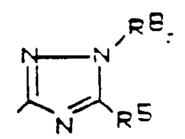
,
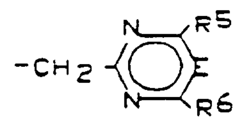
,

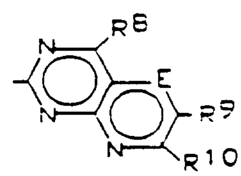
ou
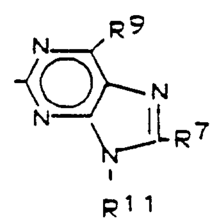

| | |
|---|---|
| E | désignant CH ou N, |
| G | O ou $CH_2$, |
| $R^5$ et $R^6$, | indépendamment l'un de l'autre, l'hydrogène, un $(C_1\text{-}C_6)$alkyle, un $(C_1\text{-}C_6)$alcoxy ou un $(C_1\text{-}C_6)$alkylthio, dont les alkyles peuvent avoir comme substituants un ou plusieurs halogènes ou un ou deux groupes $(C_1\text{-}C_4)$alcoxy ou $(C_1\text{-}C_4)$alkylthio, ou encore un radical $-NR^{12}R^{13}$, - $OCHR^7\text{-}CO_2R^{12}$, un $(C_3\text{-}C_6)$cycloalkyle, un $(C_3\text{-}C_5)$alcényle, un $(C_2\text{-}C_4)$alcynyle, un $(C_3\text{-}C_5$-alcényloxy) ou $(C_3\text{-}C_5)$alcynyloxy, |
| $R^7$ | l'hydrogène ou un $(C_1\text{-}C_4)$alkyle, |
| $R^8$ | un $(C_1\text{-}C_4)$alkyle, $-CHF_2$ ou $-CH_2F_3$, |
| $R^9$ et $R^{10}$, | indépendamment l'un de l'autre, l'hydrogène, un $(C_1\text{-}C_4)$alkyle, un $(C_1\text{-}C_5)$alcoxy ou |

un halogène, et

$R^{11}$ l'hydrogène, un ($C_1$-$C_4$)alkyle, $-CHF_2$ ou $-CH_2F_3$, et

$R^{12}$ et $R^{13}$, indépendamment l'un de l'autre, étant l'hydrogène, un ($C_1$-$C_4$)alkyle, un ($C_2$-$C_4$)-alcényle ou un ($C_3$-$C_4$)alcynyle.

**2.** Composés de formule I selon la revendication 1 et sels de ces composés, dans lesquels

n est le nombre 0 ou 1,

Y l'oxygène,

$R^1$ le groupe éthyle, propyle ou isopropyle,

$R^{2'}$ à la position 6 du cycle phénylique, désigne le fluor, le chlore, un ($C_1$-$C_4$)alkyle, un ($C_1$-$C_4$)alcoxy ou un ($C_1$-$C_4$-alcoxy)-carbonyle

$R^3$ est l'hydrogène, un ($C_1$-$C_4$)alkyle ou un ($C_3$-$C_4$)alcényle,

$R^4$ un radical de formule

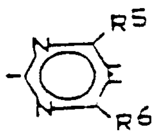

E désignant CH ou un atome d'azote, et

$R^5$ et $R^6$ étant chacun un ($C_1$-$C_4$)alkyle, un ($C_1$-$C_4$)alcoxy ou un ($C_1$-$C_4$)alkylthio, la partie alkylique des radicaux précédents pouvant avoir comme substituants un ou plusieurs atomes d'halogènes ou un ou deux groupes ($C_1$-$C_4$)alcoxy ou ($C_1$-$C_4$)alkylthio.

**3.** Composés de formule I selon la revendication 2 et sels de ces composés, caractérisés en ce que

$R^1$ est le groupe éthyle,

n le nombre 0,

Y l'oxygène,

$R^3$ l'hydrogène,

E le groupe CH, et

$R^5$ et $R^6$ sont chacun, indépendamment l'un de l'autre, le groupe méthyle ou méthoxy.

**4.** Procédé de préparation des composés de formule I ou de leurs sels selon l'une des revendications 1 à 3, procédé caractérisé en ce que :

(a) on fait réagir un composé de formule II

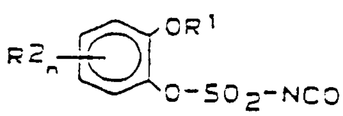

(II)

avec un composé de formule III

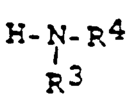

(III)

ou bien

(b) on fait réagir un composé de formule IV

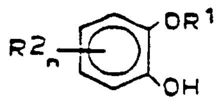

(IV)

avec une chlorofulsonylurée de formule V

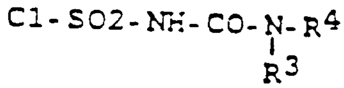

(V)

ou encore
(c) on fait réagir un composé de formule VI

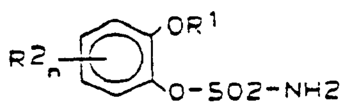

(VI)

avec un carbamate de formule VII

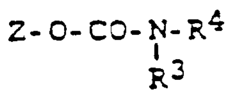

(VII)

Z désignant le groupe phényle ou un ($C_1$-$C_6$)alkyle,
et le cas échéant on transforme en sels les composés de formule I ainsi obtenus.

**5.** Produits herbicides ou régulateurs de la croissance de végétaux, caractérisés en ce qu'ils comprennent une proportion efficace d'un ou de plusieurs composés de formule I selon la revendication 1, 2 ou 3, ou de sels de ces composés, avec des matières auxiliaires inertes.

**6.** L'emploi des composés de formule I selon la revendication 1, 2 ou 3 ou de leurs sels comme herbicides ou régulateurs de la croissance de végétaux.

**7.** Procédé de lutte contre des plantes nuisibles, procédé caractérisé en ce que l'on applique sur ces plantes ou sur les sols où on les cultive une quantité appropriée d'un ou de plusieurs composés de formule I ou de leurs sels selon la revendication 1, 2 ou 3.

**8.** Procédé de régulation de la croissance de plantes utiles, procédé caractérisé en ce que l'on applique sur ces plantes ou sur les surfaces de culture une quantité appropriée d'un ou de plusieurs composés de formule I selon la revendication 1, 2 ou 3, ou de leurs sels.

**Revendications pour l'Etat contractant suivant : ES**

**1.** Procédé de lutte contre des plantes nuisibles, procédé caractérisé en ce que l'on applique sur ces plantes ou sur les sols de culture une quantité appropriée d'un ou de plusieurs composés de formule I ci-dessous ou de sels de ces composés,

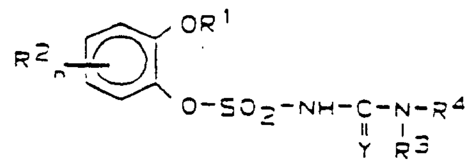

dans laquelle

| | |
|---|---|
| $R^1$ | représente le groupe éthyle, propyle ou isopropyle, |
| $R^2$ | un halogène ou un groupe $NO_2$, $CF_3$, CN, $(C_1\text{-}C_4)$alkyle, $(C_1\text{-}C_4)$alcoxy, $(C_1\text{-}C_4)$-alkylthio ou $(C_1\text{-}C_4$-alcoxy)carbonyle |
| n | le nombre 0, 1, 2 ou 3 |
| Y | l'oxygène ou le soufre, |
| $R^3$ | l'hydrogène ou bien un $(C_1\text{-}C_8)$alkyle, $(C_2\text{-}C_8)$alcényle, $(C_2\text{-}C_8)$alcynyle ou un $(C_1\text{-}C_4$-alcoxy), et |
| $R^4$ | un radical hétérocyclique de l'une des formules suivantes |

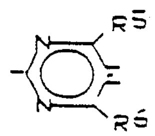 , 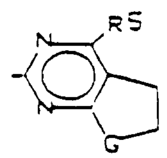 , 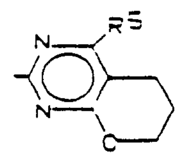 ,

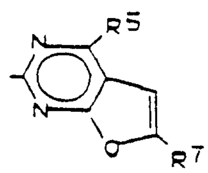 , 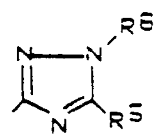 , 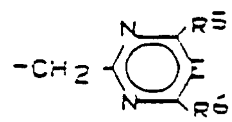 ,

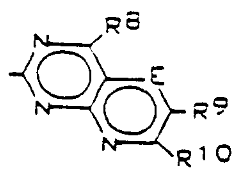 ou 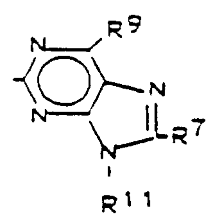

| | |
|---|---|
| E | désignant CH ou N, |
| G | O ou $CH_2$, |
| $R^5$ et $R^6$, | indépendamment l'un de l'autre, l'hydrogène, un $(C_1\text{-}C_6)$alkyle, un $(C_1\text{-}C_6)$alcoxy ou un $(C_1\text{-}C_6)$alkylthio, dont les alkyles peuvent avoir comme substituants un ou plusieurs halogènes ou un ou deux groupes $(C_1\text{-}C_4)$alcoxy ou $(C_1\text{-}C_4)$alkylthio, ou encore un radical $-NR^{12}R^{13}$, - $OCHR^7\text{-}CO_2R^{12}$, un $(C_3\text{-}C_6)$cycloalkyle, un $(C_3\text{-}C_5)$alcényle, un $(C_2\text{-}C_4)$alcynyle, un $(C_3\text{-}C_5$-alcényloxy) ou $(C_3\text{-}C_5)$alcynyloxy, |
| $R^7$ | l'hydrogène ou un $(C_1\text{-}C_4)$alkyle, |
| $R^8$ | un $(C_1\text{-}C_4)$alkyle, $-CHF_2$ ou $-CH_2F_3$, |
| $R^9$ et $R^{10}$, | indépendamment l'un de l'autre, l'hydrogène, un $(C_1\text{-}C_4)$alkyle, un $(C_1\text{-}C_5)$alcoxy ou un halogène, et |
| $R^{11}$ | l'hydrogène, un $(C_1\text{-}C_4)$alkyle, $-CHF_2$ ou $-CH_2F_3$, et |
| $R^{12}$ et $R^{13}$, | indépendamment l'un de l'autre, étant l'hydrogène, un $(C_1\text{-}C_4)$alkyle, un $(C_2\text{-}C_4)$-alcényle ou un $(C_3\text{-}C_4)$alcynyle. |

**2.** Procédé selon la revendication 1, caractérisé en ce que dans les composés de formule I :

n est le nombre 0 ou 1,

Y l'oxygène,
$R^1$ le groupe éthyle, propyle ou isopropyle,
$R^{2'}$ à la position 6 du cycle phénylique, désigne le fluor, le chlore, un $(C_1\text{-}C_4)$alkyle, un $(C_1\text{-}C_4)$alcoxy ou un $(C_1\text{-}C_4\text{-alcoxy})$-carbonyle
$R^3$ est l'hydrogène, un $(C_1\text{-}C_4)$alkyle ou un $(C_3\text{-}C_4)$alcényle,
$R^4$ un radical de formule

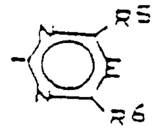

E désignant CH ou un atome d'azote, et
$R^5$ et $R^6$ étanf chacun un $(C_1\text{-}C_4)$alkyle, un $(C_1\text{-}C_4)$alcoxy ou un $(C_1\text{-}C_4)$alkylthio, la partie alkylique des radicaux précédents pouvant avoir comme substituants un ou plusieurs atomes d'halogènes ou un ou deux groupes $(C_1\text{-}C_4)$alcoxy ou $(C_1\text{-}C_4)$alkylthio.

**3.** Procédé selon la revendication 2, caractérisé en ce que dans les composés de formule I :
$R^1$ est le groupe éthyle,
n le nombre 0,
Y l'oxygène,
$R^3$ l'hydrogène,
E un groupe CH, et
$R^5$ et $R^6$ sont chacun, indépendamment l'un de l'autre, le groupe méthyle ou méthoxy.

**4.** Procédé de préparation des composés de la revendication 1, 2 ou 3, de formule I, ou de leurs sels, procédé caractérisé en ce que :
(a) on fait réagir un composé de formule II

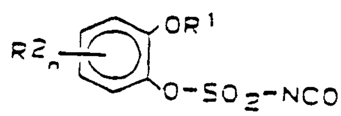

(II)

avec un composé de formule III ou bien

$$\begin{array}{l}H\text{-}N\text{-}R^4\\ \quad\;\, |\\ \quad\;\, R^3\end{array} \qquad (III)$$

(b) on fait réagir un composé de formule IV

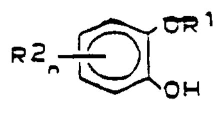

(IV)

avec une chlorofulsonylurée de formule V

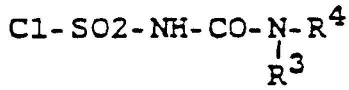

(V)

ou encore
(c) on fait réagir un composé de formule VI

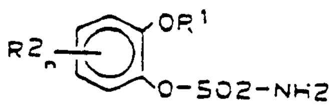

(VI)

avec un carbamate de formule VII

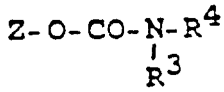

(VII)

Z désignant le groupe phényle ou un ($C_1$-$C_6$)alkyle,
et le cas échéant on transforme en sels les composés de formule I ainsi obtenus.

**5.** Procédé de régulation de la croissance de plantes utiles, procédé caractérisé en ce que l'on applique sur ces plantes ou sur les surfaces de culture une quantité appropriée d'un ou de plusieurs composés de formule I selon la revendication 1, 2 ou 3, ou de sels de ces composés.